# EUROPEAN PATENT APPLICATION

(11) **EP 1 384 999 A1**
(43) Date of publication of application: **28.01.2004**
(21) Application number: 02714580.4
(22) Date of filing: 16.04.2002
(51) Int. Cl.: G01N 33/50, G01N 33/15, G01N 33/60, G01N 33/566, G01N 33/53

(54) **BACTERIOTOXIN ADSORBENTS AND METHOD OF SCREENING THE SAME**

(30) Priority: 18.04.2001 JP 2001119547
(71) Applicant: Japan Science and Technology Corporation, Kawaguchi-shi, Saitama-ken 332-0012 (JP)
(72) Inventor: FUKASE, Koichi, Kadoma-shi, Osaka 571-0013 (JP); NESTLER, Hans Peter, 65779 Kelikheim (DE)
(74) Representative: Cresswell, Thomas Anthony
(86) International application number: PCT/JP2002/003757
(87) International publication number: WO 2002/086503

(57) **Abstract**

It is intended to provide novel lipopolysaccharide adsorbents (bacteriotoxin adsorbents) which can be easily synthesized and modified into various analogous structures and are usable in adsorbing various lipopolysaccharides and a method of screening the same. A method of screening a bacteriotoxin adsorbent characterized by performing a binding assay with a peptide library with the use of a lipid A radioisotope-labeled preparation wherein a radioisotope has been introduced into a phosphonoxyethyl derivative of biosynthetic precursor type lipid A or Escherichia coli type lipid A; and bacteriotoxin adsorbents comprising a specific peptide library which are found out by the above screening method.

## Description

### Technical Field

The present invention relates to a method for screening and preparing an endotoxin adsorbent and to an adsorbent having a specific structure found by the method.

### Background of the Invention

Higher animals have a system of activating their own immune system when bacteria invade into the body, and that system has been receiving public attention as innate immunity. Lipopolysaccharide (LPS, endotoxin) is a main component for surface layer of cells of Gram-negative bacteria such as *Escherichia coli* and salmonella. It acts as a signal for invasion of bacteria and produces various mediators such as cytokine, prostaglandin, lipid such as PAF, or NO to activate the immune system. When an appropriate amount of lipopolysaccharide acts, an advantageous immune response takes place while, when lipopolysaccharide excessively acts due to infectious diseases or the like, the immune system runs out of control and high fever, systemic blood coagulation, etc. are resulted whereby fatal shock is induced. On the other hand, interfusion of lipopolysaccharide during an artificial dialysis also has a possibility of inducing various complications by induction of cytokine as a result of introduction of lipopolysaccharide into the body during the dialysis. Further, interfusion of lipopolysaccharide in biological preparations is a big problem as well. Accordingly, there has been a demand for development of lipopolysaccharide adsorbent to remove the lipopolysaccharide. Up to now, however, it is only Toraymyxine (trade name; Toray), where polymyxin B, an antibiotic, is immobilized with fiber which is used for an endotoxin adsorption therapy, that has been developed and actually used as an adsorbent for lipopolysaccharide.

### Disclosure of the Invention

The invention has been achieved in view of the above-mentioned current situation and its object is to provide a novel lipopolysaccharide adsorbent (endotoxin adsorbent) which can be easily synthesized, can be easily adjusted various analogous structures easily, and can be used for adsorption of various kinds of lipopolysaccharides, and a method for screening the same.

The invention relates to a method for screening an adsorbent for endotoxin characterized in that a binding assay with a peptide library is conducted using a lipid A radioisotope-labeled preparation where radioactive element is introduced into a phosphonoxyethyl derivative of a lipid A of a biosynthetic precursor type or a lipid A of an *Escherichia coli* type and also to an adsorbent for endotoxin which is found by the screening method.

The invention also relates to a peptide library where a solid phase carrier having a functional group is bound to a compound, having a functional group which is able to bind to the said functional group and having 2 or more other functional groups, and is further bound to an oligopeptide.

The invention further relates to a peptide library where a solid carrier having a functional group is bound to a compound, having a functional group which is able to bind to the said functional group and having 2 or more other functional groups, and is further bound to a compound, having a functional group which is able to bind to the said other functional groups and having 2 or more still other functional groups and, after that, an oligopeptide is bound thereto.

The invention still further relates to a lipid A radioisotope-labeled preparation where radioactive element is introduced into a phosphonoxyethyl derivative of a lipid A of a synthetic precursor type or a lipid A of an *Escherichia coli* type.

Thus it has been clarified that, although a glycosylphosphate group at position 1 of the lipid A of a natural type is chemically unstable, a PE analog where the group is substituted with a phosphonoxyethyl (PE) group is chemically stable showing the same activity as that of a natural type. The present inventors already reported an efficient synthetic method for the PE analog (*Bull. Chem. Soc. Jpn*., 72, 1377 (1999)) and, at this time, they have succeeded in the synthesis of *Escherichia coli* lipid A tritium-labeled PE analog using NaB³H₄ for the reduction of an aldehyde intermediate in the synthesis of PE analog, and have found that, when a binding assay with a specific peptide library is carried out using the radioisotope-labeled preparation, an effective screening of adsorbents for endotoxin can be conducted. Accordingly, the invention has been achieved.

### Brief Description of the Invention

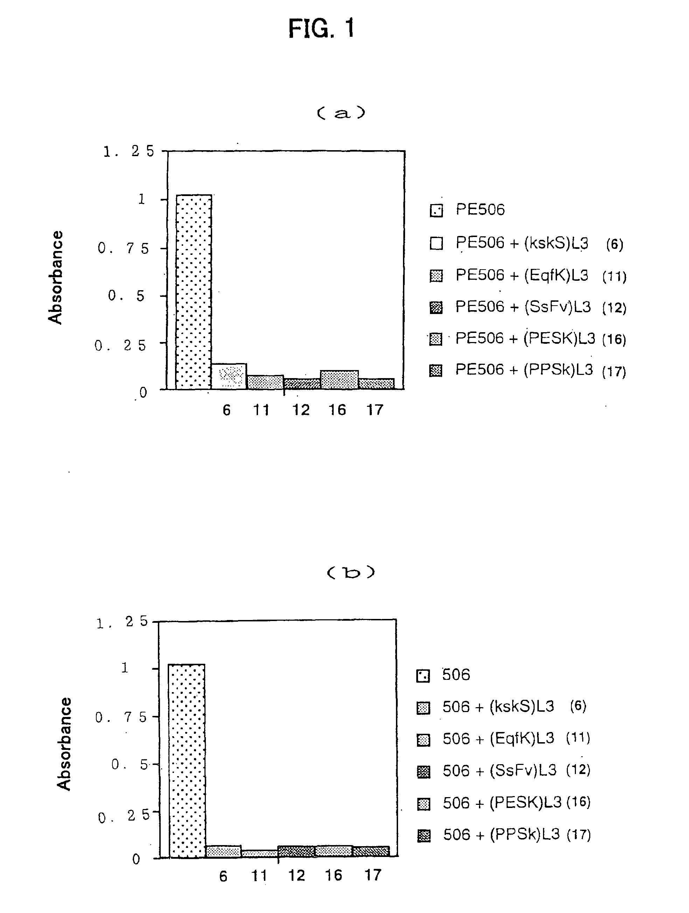

Fig. 1 shows the result where a binding ability of the peptide library of the invention (using Tenta Gel which is a polyethylene glycol-polystyrene resin as a solid phase carrier) to lipid A, lipid A analog and lipopolysaccharide was investigated.

In Fig. 1, (a) is a result of the binding assay with a phosphonoxyethyl derivative of an *Escherichia coli* type lipid A (PE 506), (b) is that with lipid A of an *Escherichia coli* type (506), (c) is that with a phosphonoxyethyl derivative of lipid A of a biosynthetic precursor type (PE 406), (d) is that with lipid A of a biosynthetic precursor type (406), (e) is that with lipopolysaccharide of an *Escherichia coli* Re mutant (ReLPS) and (f) is that with an *Escherichia coli* lipopolysaccharide (LPS).

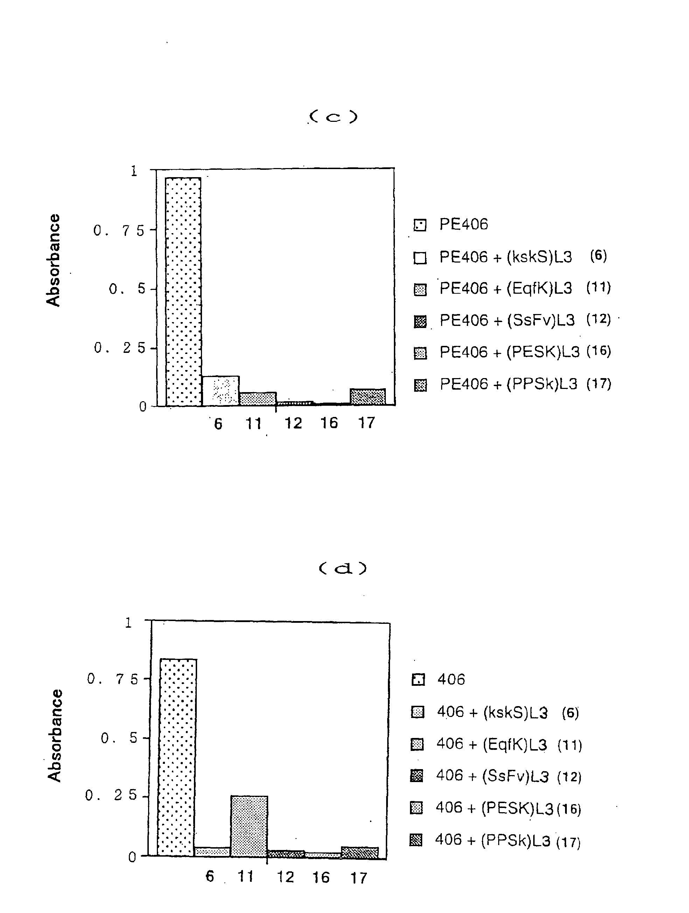

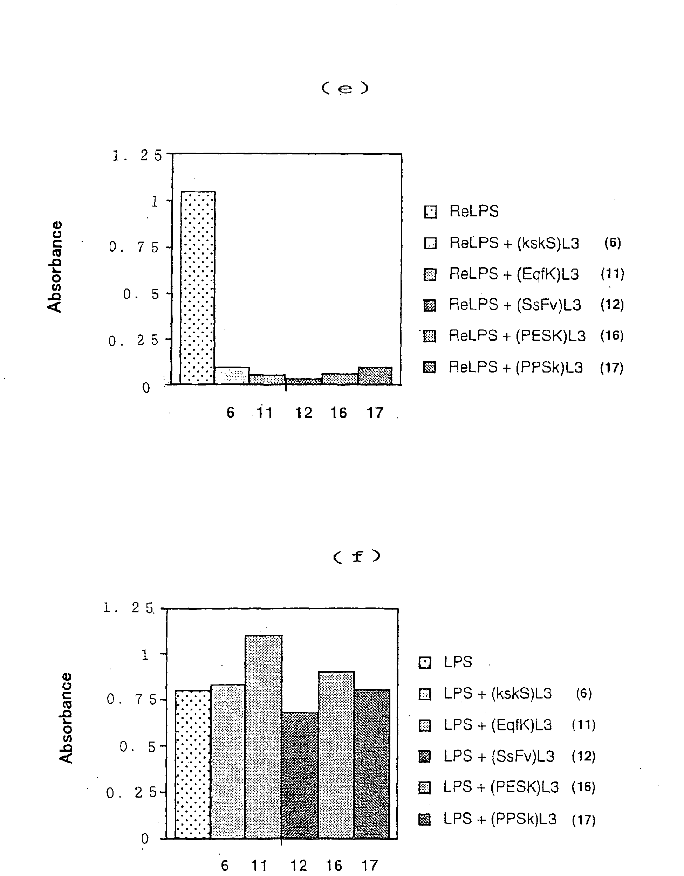

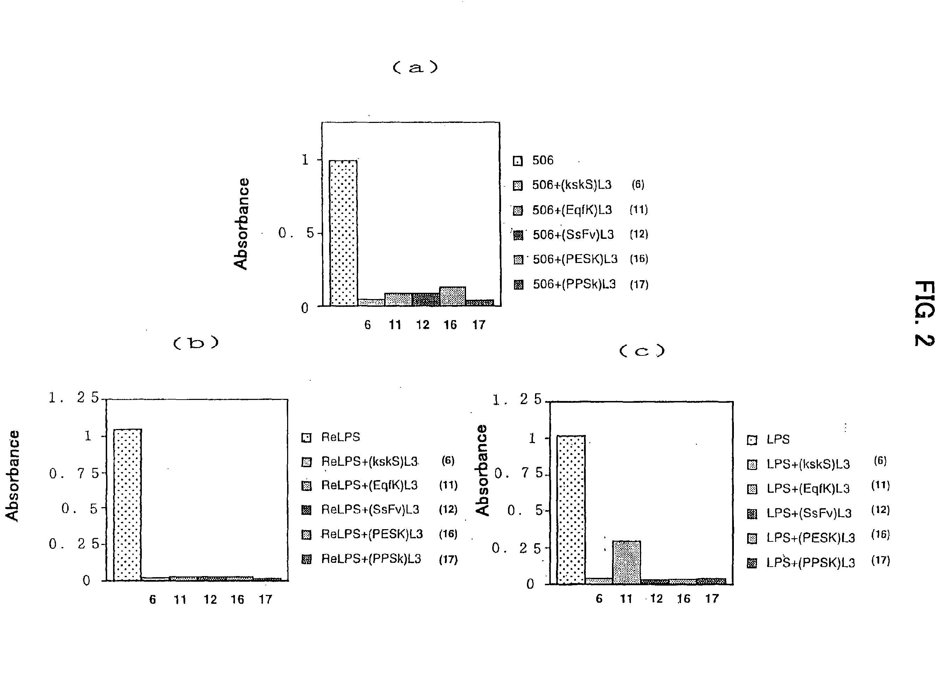

Fig. 2 shows the result where a binding ability of the peptide library of the invention (using TOYOPEARL which is a hydrophilic vinyl polymer as a solid phase carrier) to lipid A, lipid A analog and lipopolysaccharide was investigated.

In Fig. 2, (a) is a result of the binding assay with lipid A of an *Escherichia coli* type (506), (b) is that with a lipopolysaccharide of an *Escherichia coli* Re mutant (ReLPS) and (c) is that with an *Escherichia coli* lipopolysaccharide (LPS).

### Best Mode for Carrying Out the Invention

Tritium (³H) is exemplified as a radioactive element in a radioisotope-labeled lipid A analog where radioactive element is introduced into a phosphonoxyethyl derivative of lipid A of a biosynthetic precursor type or lipid A of an *Escherichia coli* type used in the invention.

What is represented by the following formula [4] is exemplified as a radioisotope-labeled lipid A analog where radioactive element is introduced into a phosphonoxyethyl derivative of lipid A of an *Escherichia coli* type used in the invention.

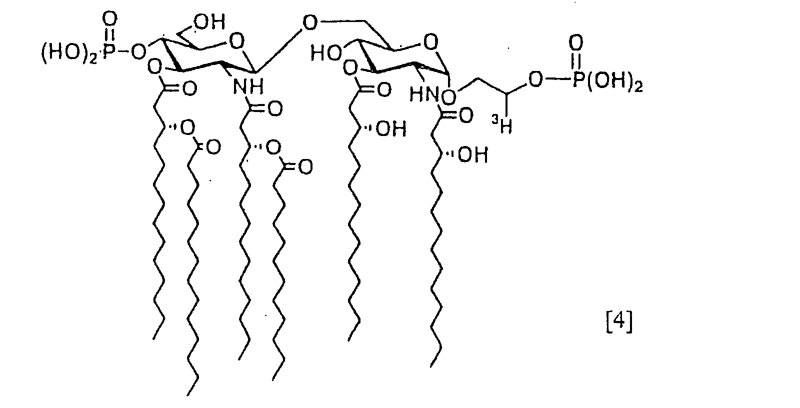

What is represented by the following formula [5] is exemplified as a radioisotope-labeled lipid A analog where radioactive element is introduced into a phosphonoxyethyl derivative of lipid A of a biosynthetic precursor type used in the invention.

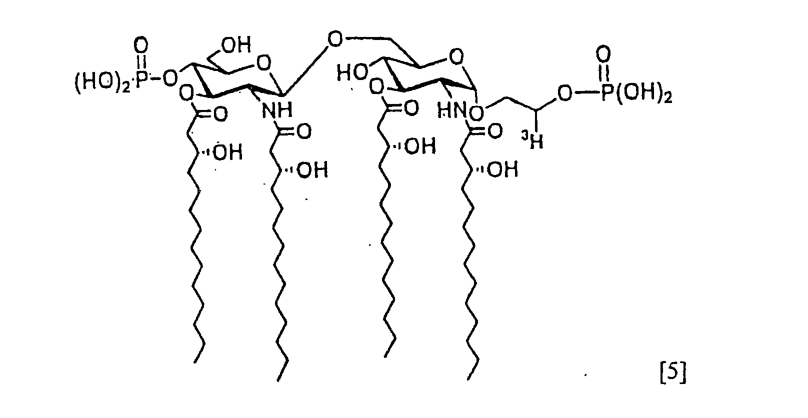

A process for producing the above-mentioned radioisotope-labeled lipid A analog will be illustrated in detail in the following Examples.

The peptide library used in the invention includes a peptide library which is prepared by binding a solid phase carrier or a soluble carrier having a functional group to a compound having a functional group which is able to bind to the said functional group and also having 2 or more other functional groups followed by binding an oligopeptide thereto.

A peptide library prepared by binding a solid phase carrier or a soluble carrier having a functional group to a compound, having a functional group which is able to bind to the said functional group and also having 2 or more other functional groups, then binding to a compound, having a functional group which is able to bind to the said other functional groups and also having 2 or more still other functional groups, and further binding to an oligopeptide thereto is also preferable.

The solid phase carrier or the soluble carrier having a functional group includes a solid phase carrier or a soluble carrier having an amino group.

When the solid phase carrier or the soluble carrier having a functional group is a solid phase carrier or a soluble carrier having an amino group, the compound, having a functional group which is able to bind to the amino group and also having 2 or more other functional groups, includes a basic amino acid represented by the formula HOOC(NH₂)CH(CH₂)ₙNH₂ (in the formula, n is an integer of 1 to 4), and a carboxylic acid having 2 or more hydroxyl groups such as deoxycholic acid and chenodeoxycholic acid.

With regard to the specific examples of the above amino acid, lysine and omithine may be listed as preferred ones.

When the solid phase carrier or the soluble carrier having a functional group is a solid phase carrier or a soluble carrier having an amino group, and the compound, having a functional group which is able to bind to the said amino group and also having two or more other functional groups, is an amino acid represented by the formula HOOC(NH₂)CH(CH₂)ₙNH₂ (in the formula, n is an integer of 1 to 4), the compound, having a functional group which is able to bind to the said other functional groups and having two or more still other functional groups, includes a basic amino acid represented by the formula HOOC(NH₂)CH(CH₂)ₙNH₂ (in the formula, n is an integer of 1 to 4).

With regard to the specific examples of the above amino acid, there may be exemplified lysine and ornithine as preferred ones.

In the peptide library used in the invention, an oligopeptide having 2 to 20 amino acid residues, preferably 3 to 10, more preferably, 4 to 8 may be exemplified as an oligopeptide which is to be bound to the solid phase carrier or the soluble carrier having a functional group via a compound having a functional group which is able to bind to the said functional group or having two or more other functional groups, or via a compound where the solid phase carrier or the soluble carrier having a functional group is bound to a compound having a functional group which is able to be bound to the said functional group and two or more other functional groups and further bound to a compound having a functional group which is able to be bound to the said other functional groups and having two or more still other functional groups.

Although there is no particular limitation for the type of the amino acid residue, there may be exemplified Gly, D,L-Val, D,L-Phe, D,L-Pro, D,L-Ser, D,L-Gln, D,L-Glu, D,L-Lys, etc. as preferred ones.

A specific example for the peptide library concerning the invention includes a compound represented by the following formula [1], [2] or [3]. 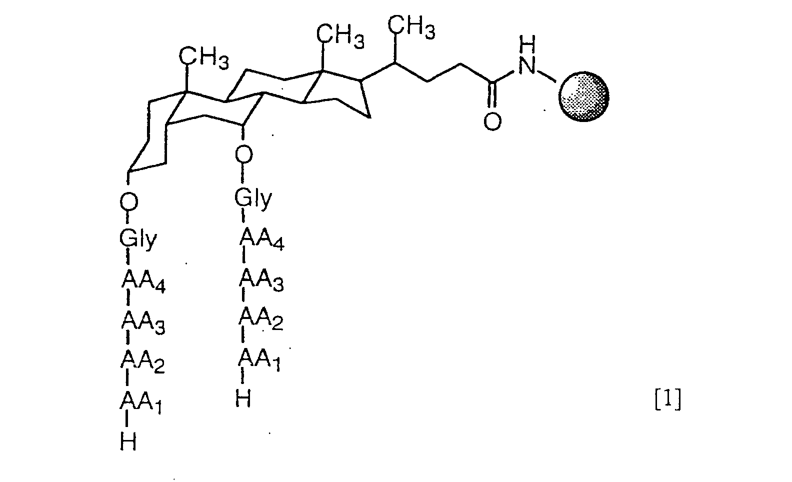 (in the formula, a spherical part represents a solid phase carrier or a soluble carrier, and AA₁, AA₂, AA₃ and AA₄ each independently represents an amino acid residue.) 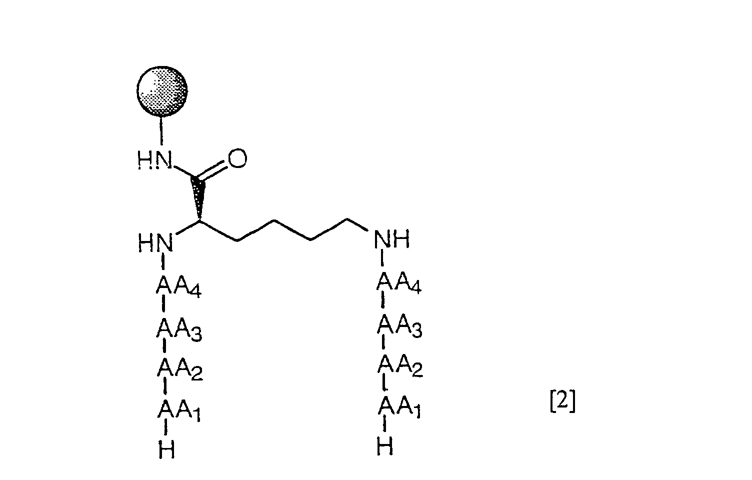 (in the formula, a spherical part represents a solid phase carrier or a soluble carrier, and AA₁, AA₂, AA₃ and AA₄ each independently represents an amino acid residue.) 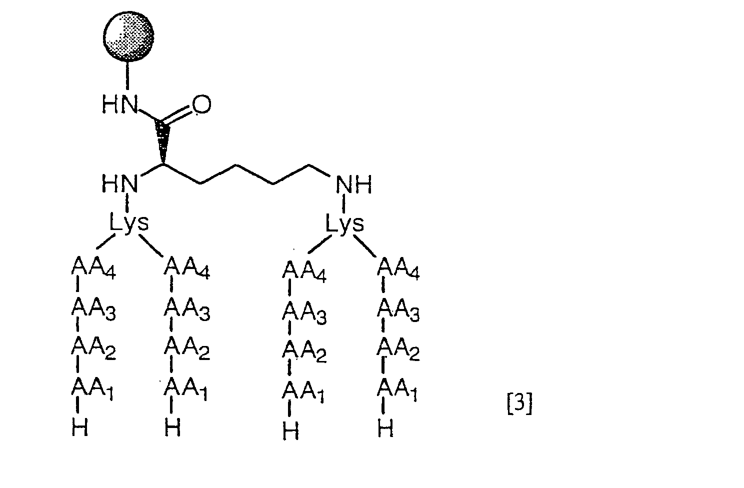 (in the formula, a spherical part represents a solid phase carrier or a soluble carrier, and AA₁, AA₂, AA₃ and AA₄ each independently represents an amino acid residue.)

In the peptide library represented by the above formula [1], [2] or [3], there is no particular limitation for the type of the amino acid residue represented by AA₁, AA₂, AA₃ and AA₄, and any amino acid residue may be used. The examples of the preferred ones are Gly, D,L-Val, D,L-Phe, D,L-Pro, D,L-Ser, D,L-Gln, D,L-Glu and D,L-Lys.

The endotoxin adsorbent of the invention can be detected and extracted by means of a binding assay to the above peptide library using the above radioisotope-labeled lipid A preparation. The specific examples of the endotoxin adsorbent of the invention includes such as those where AA₁, AA₂, AA₃ and AA₄ in the formula [1] comprise any of the combinations mentioned in the following Table 1

**Table 1**

| AA1 | AA2 | AA3 | AA4 |
|---|---|---|---|
| D―Phe | D―Val | L―Gln | D―Phe |
| L―Lys | D―Ser | D―Val | D―Gln |
| D―Pro | D―Phe | L―Phe | L―Val |
| D―Gln | D―Gln | L―Phe | D―Lys |
| D―Val | L―Pro | D―Ser | D―Ser |

those where AA₁, AA₂, AA₃ and AA₄ in the formula [2] comprise any of the combinations mentioned in the following Table 2.

**Table 2**

| AA1 | AA2 | AA3 | AA4 |
|---|---|---|---|
| D―Pro | L―Glu | D―Glu | L―Val |
| D―Val | D―Pro | D―Lys | L―Gln |
| D―Val | L―Glu | L―Val | D―Glu |
| L―Val | D―Pro | D―Val | D―Ser |
| L―Val | D―Pro | L―Phe | Gly |
| D―Lys | L―Glu | D―Glu | L―Gln |
| L―Lys | D―Lys | D―Lys | D―Ser |
| L―Phe | L―Lys | Gly | L―Ser |
| D―Gln | D―Glu | D―Ser | L―Glu |

,or those where AA₁, AA₂, AA₃ and AA₄ in the formula [3] comprise any of the combinations mentioned in the following Table 3.

**Table 3**

| AA1 | AA2 | AA3 | AA4 | AA1 | AA2 | AA3 | AA4 |
|---|---|---|---|---|---|---|---|
| D-Lys | D-Ser | D-Lys | L-Ser | D-Val | L-Glu | L-Val | L-Lys |
| L-Gln | D-Lys | L-Glu | L-Gln | D-Pro | L-Ser | D-Glu | L-Lys |
| D-Ser | L-Phe | D-Gln | L-Lys | D-Ser | L-Ser | L-Lys | D-Lys |
| L-Phe | D-Lys | L-Gln | L-Lys | L-Lys | L-Lys | D-Lys | D-Lys |
| L-Lys | L-Lys | L-Gln | L-Lys | L-Gln | L-Glu | L-Lys | D-Lys |
| L-Val | L-Val | L-Lys | L-Lys | L-Lys | L-Gln | L-Gln | D-Glu |
| L-Gln | D-Lys | Gly | L-Lys | L-Lys | L-Ser | L-Lys | L-Glu |
| L-Glu | D-Gln | D-Phe | L-Lys | D-Gln | D-Val | D-Lys | L-Glu |
| D-Gln | D-Glu | L-Phe | L-Lys | D-Lys | L-Lys | D-Glu | L-Glu |
| L-Ser | D-Ser | L-Phe | D-Val | L-Lys | D-Val | L-Glu | D-Glu |
| D-Pro | L-Ser | L-Pro | L-Val | L-Lys | L-Ser | L-Val | Gly |
| D-Pro | L-Pro | D-Ser | L-Phe | L-Lys | D-Gln | L-Gln | L-Lys |
| L-Pro | L-Pro | L-Ser | L-Val | D-Val | L-Pro | L-Phe | L-Lys |
| L-Pro | L-Val | L-Gln | D-Gln | L-Pro | D-Pro | D-Phe | L-Lys |
| Gly | D-Val | L-Lys | Gly | L-Pro | L-Glu | L-Ser | L-Lys |
| L-Phe | L-Val | L-Glu | D-Ser | L-Phe | D-Val | D-Glu | L-Lys |
| L-Phe | L-Gln | L-Lys | L-ser | L-Gln | D-Gln | L-Gln | D-Lys |
| D-Lys | L-Gln | L-Pro | D-Phe | L-Pro | L-Pro | L-Ser | D-Lys |
| D-Lys | D-Lys | D-Val | D-Gln | | | | |

Although the correlation between the oligopeptide structure and its efficacy as an endotoxin adsorbent has not been fully clarified yet, it is likely that the structure where there is lysine in AA₄ is more effective.

The solid phase carrier or the soluble carrier having a functional group used in the invention includes, for example, resin or polyethylene glycol having functional group such as amino group, carboxyl group and hydroxyl group and, preferably, resin in beads. Specific examples of such resin are a polyethylene glycol-polystyrene resin having the above-mentioned functional group [examples of the specific trade name are Tenta Gel S-NH₂ (Rapp Polymere), Aminomethyl Nova Gel HL (Novabiochem), etc.], a hydrophilic vinyl polymer [examples of the specific trade name are TSK gel AF-Amino TOYOPEARL 650 S (Tosoh)], etc.].

As hereunder, outline of the binding assay of a radioisotope-labeled lipid A analog with a molecular tweezers library (peptide library) will be mentioned.

### Preparation of molecular tweezers library

First, an encode molecular tweezers library (peptide library) was prepared by a split synthesis.

Namely, chenodeoxycholic acid was used as a template and tetrapeptide library was bound to two hydroxyl groups via Gly residue (diarm L1), tetrapeptide library was bound to each of two amino groups of L-Lys (diarm L2) and L-Lys was bound to each of amino groups of L-Lys and tetrapeptide library was bound to 4 amino groups (tetraarm L3). As a solid carrier, Tenta Gel which was a polyethylene glycol-polystyrene resin which was swollen in water was used taking the fact that an assay will be conducted in water into consideration.

When L1 was used as a core structure, deoxycholic acid was first introduced into a solid phase carrier and, after that, Fmoc-Gly was bound to two hydroxyl groups. Lysine (L2) and trilysine (L3) cores were prepared by a common peptide solid phase synthetic method. For the preparation of a library, 15 kinds of amino acids (Gly, D and L-Val, Phe, Ser, Gln, Glu, and Lys and Pro) were used. A split coupling cycle in four stages was carried out, and a library containing 3 × 15⁴ = 151,875 kinds of peptide was constructed.

### Binding assay of radioisotope-labeled lipid A analog with molecular tweezers library

The radioisotope-labeled lipid A analog represented by the above formula [4] (hereinafter, abbreviated as [³H]PE 506) or the radioisotope-labeled lipid A analog represented by the above formula [5] (hereinafter, abbreviated as [³H]PE 406) was used and a binding assay to a molecular tweezers library was carried out. Incidentally, with regard to a binding assay to a molecular tweezers library using a radioisotope-labeled compound, Nestler established it already and the means was applied (Nestler H. P.; Wennemers H.; Sherlock R.; Dong D.L.-Y *Bioorg. Med. Chem Lett*., 6, 1327 (1996).).

With regard to a binding assay using [³H]PE 506, the library (15.7 mg; containing about 19,000 resin in beads) was shaken at 4°C for 41 hours in the presence of [³H]PE 506. The resin which was bound to the molecular tweezers recognizing [³H]PE 506 according to this operation adsorbs [³H]PE 506 and, therefore, it shows radioactivity. When this was treated with a Kodak autoradiographic emulsion, the area around the resin having radioactivity was exposed. After fixing, an excessive emulsion was removed, and the resin showing radioactivity was stained in black. The resin was taken out and a peptide sequence on the resin was determined by an encode method which will be mentioned later. As a result, 19 kinds of molecular tweezers binding to [³H]PE 506 were found (Table 4).

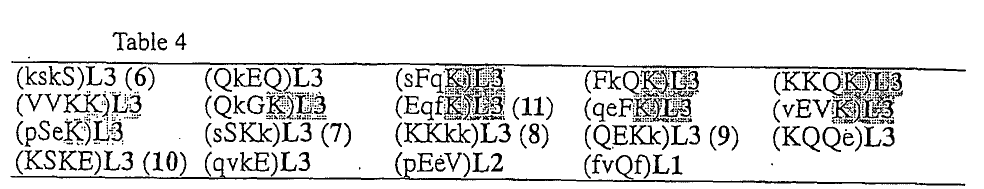

Small letters show D-amino acids while capital letters show L-amino acids.

As is apparent from Table 4, 17 kinds of molecular tweezers out of 19 had the structure of L3. In addition, at C-terminal of each arm, 9 kinds of substances having L-lysine (K) residue (K-L3 structure) and 3 kinds of substance having D-lysine (k) (k-L3 structure) were found. Although 151,875 molecular tweezers are contained in the whole library, the number of resin in beads was about 19,000 used in the actual assay and the number of molecular tweezers examined by the assay was less than 20%. Accordingly, it is predicted that, besides the peptide sequences found at this time, there will exist many sequences which bind to [³H]FE 506.

With regard to the binding assay using [³H]PE 406, the same binding assay using a library (24.2 mg; containing about 29,000 resin in beads) was carried out. As a result, 32 kinds of molecular tweezers were found (Table 5).

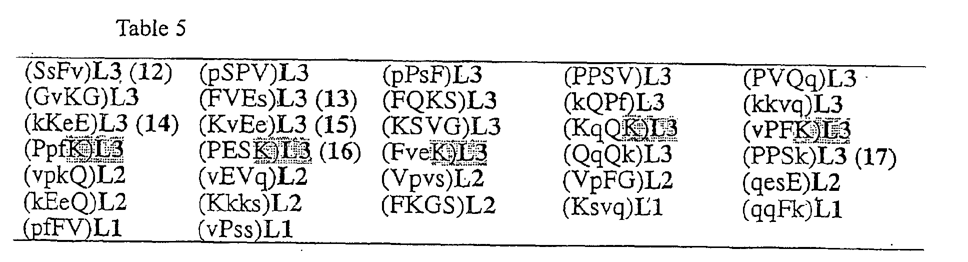

Small letters show D-amino acids while capital letters show L-amino acids.

As is apparent from Table 5, 20 kinds out of 32 had an L3 structure and, only in 5 kinds thereof, L-lysine residue was present at C-terminal of the tetrapeptide. Although it is true that this part is a consensus sequence, mobility of the conformation increased in [³H]PE 406 and, therefore, it is likely that various molecular tweezers are able to be bound.

### Re-synthesis of molecular tweezers recognizing a lipid A analog

In order to confirm whether the above molecular tweezers bind to natural type lipid A and lipopolysaccharide or not, some were selected from the above molecular tweezers and resins 6 to 17 to which the molecular tweezers were bound were prepared.

### Molecular tweezers found in a binding assay to [³H]PE 506

(k-s-k-S)₄ L 3 -TentaGel( 6 -TentaGel), (s-S-K-k)₄ L 3 -TentaGel ( 7 -TentaGel), (K-K-k-k)₄ L 3 -TentaGel( 8 -TentaGel), (Q-E-K-k)₄ L 3 -Tenta-gel( 9 -TentaGel), (K-S-K-E)₄ L 3 -TentaGel( 1 0 -TentaGel), (E-q-f-K)₄ L 3 -TentaGel( 1 1-TentaGel)

### Molecular tweezers found in a binding assay to [³H]PE 406

(S-s-F-v)₄ L 3 -TentaGel( 1 2 -TentaGel), (F-V-E-s)₄ L 3 -TentaGel( 1 3 -TentaGel), (k-K-e-E)₄ L 3 -TentaGel( 1 4 -TentaGel), (K-v-E-e)₄ L 3 -TentaGel( 1 5 -TentaGel), (P-E-S-K)₄ L 3 -TentaGel( 1 6 -TentaGel), (P-P-S-k)₄ L 3 -TentaGel( 1 7 -TentaGel)

### Binding assay using the re-synthesized molecular tweezers

Next, using the re-synthesized molecular tweezers, the binding assays were carried out to phosphonoxyethyl derivatives of lipid A of an *Escherichia coli* type (hereinafter, abbreviated as PE 506), lipid A of an *Escherichia coli* type (hereinafter, abbreviated as 506), phosphonoxyethyl derivative of lipid A of a biosynthetic precursor type (hereinafter, abbreviated as PE 406), lipid A of a biosynthetic precursor type (hereinafter, abbreviated as 406), lipopolysaccharide of Re mutant of *Escherichia coli* (ReLPS) and lipopolysaccharide of *Escherichia coli* (*E. coli* O111:B4 LPS (Sigma)). ReLPS has a structure where two residues of the sugar called Kdo are bound to a lipid A moiety lacking in most. of core polysaccharide and O-specific polysaccharide.

For reference, structural formula of ReLPS is shown below.

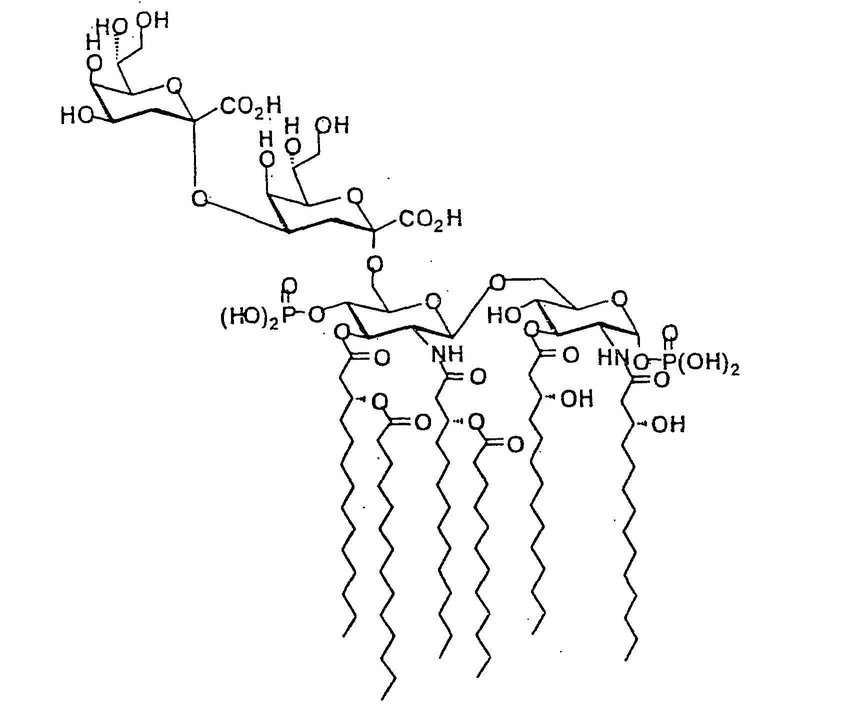

First, trifluoroacetates of molecular tweezer resins 6 to 17-Tenta Gel were used and binding assays to PE 506 and 506 were carried out. Binding to the resin was confirmed by a TLC analysis of the solution after the assay. The result is shown in Table 6.

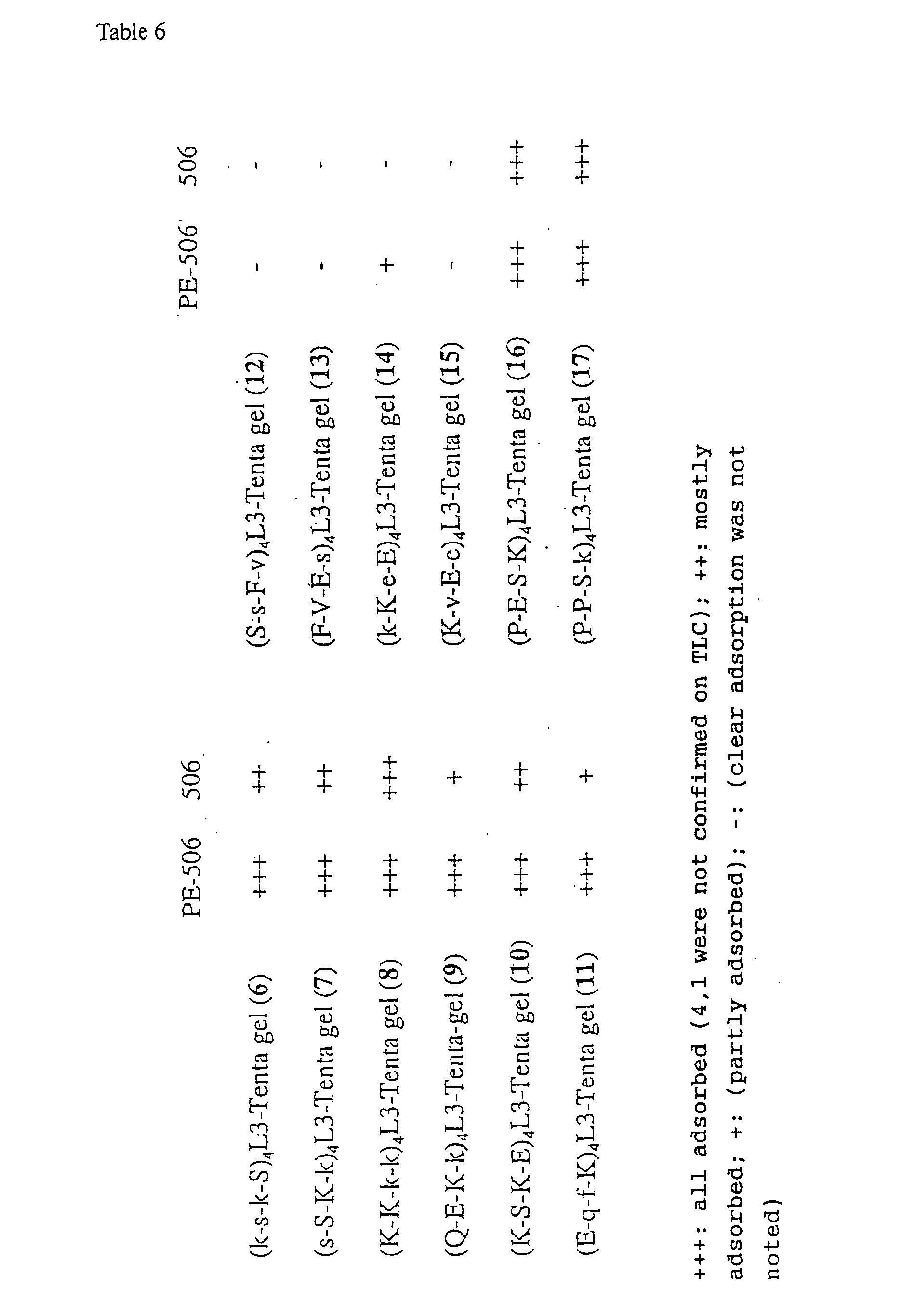

After that, adsorbing ability of the resins 6-Tenta Gel, 11-Tenta Gel, 12-Tenta Gel, 16-Tenta Gel and 17-Tenta Gel was confirmed by measuring a Limulus activity of the solution after the assay. The Limulus activity test is a method where using the reaction that lipopolysaccharide activates a coagulation enzyme of LAL (Limulus Amebocyte Lysate) which is a component extracted from blood corpuscles of horseshoe crab *(Limulus polyphemus*, etc.), endotoxin activity is subjected to a colorimetric determination.

Binding assays to 6-Tenta Gel, 11-Tenta Gel, 12-Tenta Gel, 16-Tenta Gel and 17-Tenta Gel was carried out same as above using PE 506, 506, PE 406, 406, ReLPS and *Escherichia coli* lipopolysaccharide (*E. coli* O111:B4 LPS (Sigma)) and Limulus activity of the supernatant liquid was measured. When such an endotoxin was adsorbed with the resin, the observed absorbance lowers. In addition, since each resin was washed with water before conducting the assay here, it is likely that most of the amino groups on the resin were in a free state. Therefore, adsorbing ability of the resin to lipopolysaccharide and lipid A which are acidic complex carbohydrates is improved before washing with water. Actually, when the above-mentioned trifluoroacetate was used, 12-Tenta Gel did not adsorb PE 506 and 506 while 12-Tenta Gel after being washed with water well adsorbed both of them. The resin 11-Tenta Gel had a weak adsorbing ability to 406. Although all resins showed strong adsorbing ability to natural ReLPS, they hardly showed adsorbing ability to *Escherichia coli* lipopolysaccharide (*E. coli* O111:B4 LPS (Sigma)) (refer to Fig. 1).

Tenta Gel is a graft polymer of polyethylene glycol with polystyrene and, although it is swollen in water, it is a resin having a high hydrophobic property. The *Escherichia coli* lipopolysaccharide (LPS) has a long sugar chain and it was presumed that the hydrophilic sugar chain disturbed permeation into the resin. Therefore, using TSK gel AF-Amino TOYOPEARL 650 S (Tosoh) having a high hydrophilic property as a carrier, 6-Toyopearl, 11-Toyopearl, 12-Toyopearl, 16-Toyopearl and 17-Toyopearl, to which molecular tweezers 6, 11, 12, 16 and 17 were bound, were synthesized and binding assays to 506, ReLPS and *Escherichia coli* lipopolysaccharide (*E. coli* O111:B4 LPS (Sigma)) were conducted whereupon the binding ability was evaluated by a Limulus activity. As a result, as being expected, resins where Toyopearl was used as a base efficiently adsorbed all of them with a few exceptions (refer to Fig. 2).

From the above, both 19 kinds of the molecular tweezers (peptide library) mentioned in Table 4 and 32 kinds of the molecular tweezers (peptide library) mentioned in Table 5 are greatly expected as those which are more effectively used as endotoxin adsorbents.

As compared with the known adsorbents, the adsorbent of the invention uses a natural substance (natural amino acid), which is a big advantage.

Incidentally, in order to prepare the specific adsorbent in accordance with the invention, there was prepared a library comprising 15⁴ × 3 = 151,875 being selected from 15 amino acid, further, and actually comprising 19,000 and 29,000 was prepared whereupon effective 51 adsorbents were obtained therefrom.

At that time, the selection of the 15 amino acids and the narrowing-down from 151,875 to 19,000 and 29,000 were carried out taking hydrophobic property, hydrophilic property, acidity, basicity, etc. into consideration.

### Examples

The invention will be illustrated in more detail by the following Examples although the invention is not limited by those Examples at all.

### Example 1 Synthesis of radioisotope-labeled lipid A analogs

Two kinds of radioisotope-labeled lipid A analogs were synthesized according to the following synthetic scheme.

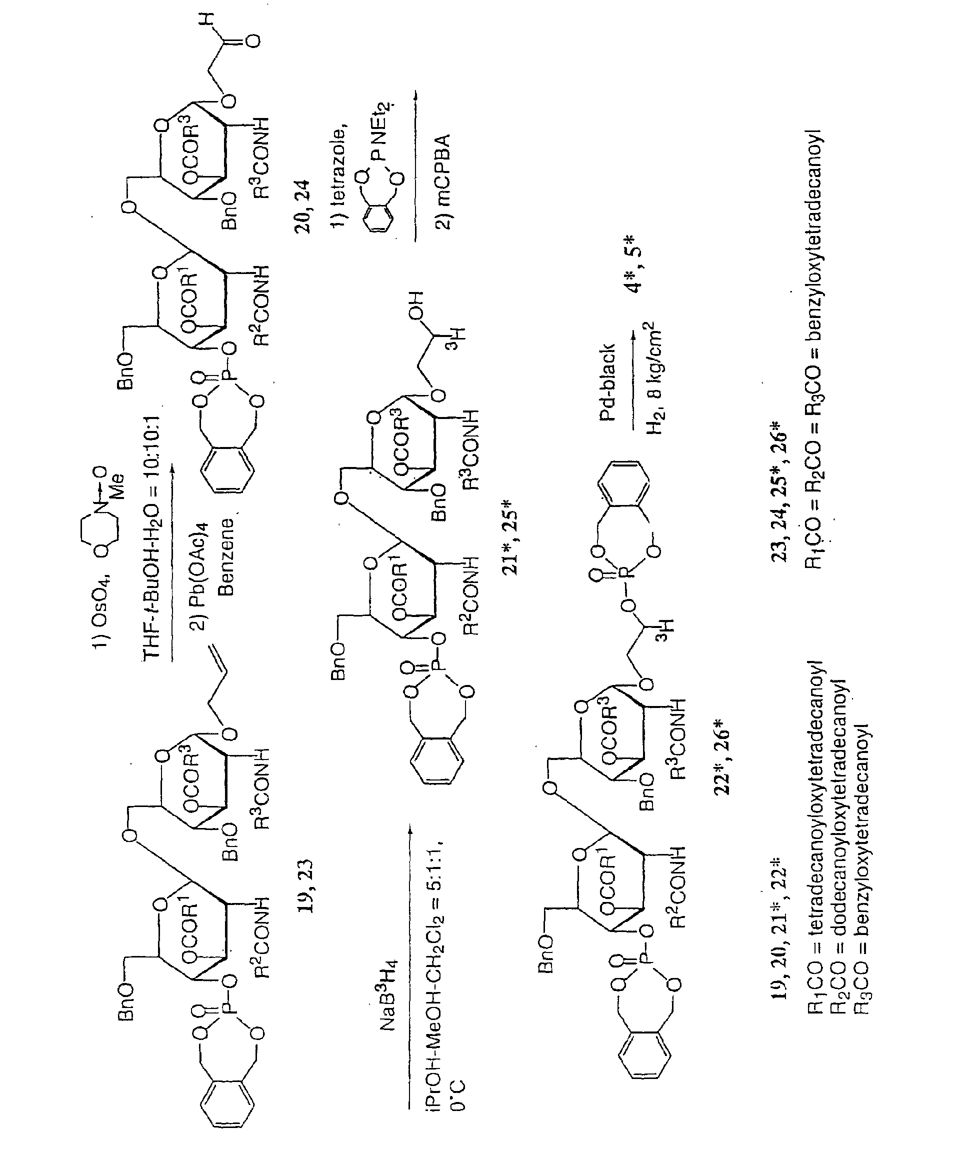

Kiesel gel 60 (E. Merck; 0.040 to 0.063 mm) was used for the silica gel column chromatography. Sephadex LH-20 was purchased from Pharmacia Biotech, Sweden. Anhydrous CH₂Cl₂ and CHCl₃ were prepared by distillation using calcium hydride as a dehydrating agent. Anhydrous tetrahydrofuran (THF) and anhydrous benzene were purchased from Kanto Kagaku K. K. NaB³H₄ (specific activity: 222 GBq mmol⁻¹) was purchased from Amersham Life Science. In an autoradiography, an imaging plate BAS-TR 2040S (Fuji Photo-Film) was used and, for the detection, a bio-imaging analyzer BAS-1500 MAC (Fuji Photo-Film) was used.

### (1) Synthesis of 2-(phosphonoxy)[2-³H₁]ethyl 2-deoxy- 6-O-[2-deoxy-2-[(R)-3-(dodecanoyloxy)tetradecanoylamino]-4-O-phosphono-3-O-[(R)-3-(tetradecanoyloxy) tetradecanoyl]-β-D-glucopyranosyl]-3-O-[(R)-3-hydroxytetradecanoyl]-2-[(R)-3-hydroxytetradecanoylamino]-α-D-glucopyranoside ([³H] PE 506: 4*)

### (1-1) Synthesis of formylmethyl 4-O-benzyl-6-O-[6-O- benzyl-2-deoxy-4-O-(1,5-dihydro-3-oxo-3λ⁵-3H-2,4,3-benzodioxaphosphepin-3-yl)-2-[(R)-3-(dodecanoyloxy)tetradecanoylamino]-3-O-[(R)-3-(tetradecanoyloxy)tetradecanoyl]-β-D -glucopyranosyl]-3-O-[(R)-3-(benzyloxy)tetradecanoyl]-2-[(R)-3-(benzyloxy) tetradecanoylamino]-2-deoxy-α-D-glucopyranoside (Compound 20)

An aqueous solution (2.5%, 400 mL, 40 mmol) of 4-methylmorpholine N-oxide (NMO) (94 mg, 0.80 mmol) and osmium tetraoxide (OsO₄) was added to a solution of the compound 19 (450 mg, 202 mmol) in THF/tert-butanol/water (10:10:1, 12 ml) with vigorous stirring. After 6 hours, a saturated aqueous solution of Na₂S₂O₃ (50 mL) was added followed by extracting with ethyl acetate (50 mL). The organic layer was washed with an aqueous solution of Na₂S₂O₃ (2 × 50 mL) and a saturated saline solution (20 mL), dried over Na₂SO₄ and concentrated *in vacuo* to obtain a diol as a crude product (458 mg). The diol was dissolved in anhydrous benzene (10 mL) and lead tetraacetate (Pb(OAc)₄) (purity: 90%, 119 mg, 242 mmol) was added thereto followed by stirring for 30 minutes. The reaction mixture was charged to a silica gel column (3 g) and eluted with ethyl acetate. The eluate was concentrated *in vacuo* and the residue was purified by a medium-pressure silica gel chromatography (20 g, toluene/ethyl acetate = 5:1) to obtain the compound 20 as a colorless syrup (377 mg, yield: 84%).
FAB-MS (positive) *m*/*z* 2244 [(M+Na)⁺]; ¹H NMR (500MHz,CDCl₃) δ =9.37(s,1H,CHO).

Synthesis of the compound 19 was carried out in accordance with a method mentioned in the literature [Liu W-C, Oikawa M, Fukase K, Suda Y, Kusumoto S. *Bull. Chem. Soc. Jpn*. 1999; 72: 1377-1385.; Fukase K, Oikawa M, Suda Y, Liu W-C, Fukase Y, Shintaku T, Sekljic H, Yoshizaki H, Kusumoto S. *J Endotoxin Res.* 1999; 5: 46-51.].

### (1-2) Synthesis of 2-hydroxy-[2-³H₁]ethyl 4-O-benzyl-6-O-[6-O-benzyl-2-deoxy-4-O-(1,5-dihydro-3-oxo-3λ⁵-3H-2,4,3-benzodioxaphosphepin-3-yl)-2-[(R)-3-(dodecanoyloxy)tetradecanoylamino]-3-O-[(R)-3-(tetradecanoyloxy) tetradecanoyl]-β-D-glucopyranosyl]-3-O-[(R)-3-(benzyloxy)tetradecanoyl]-2-[(R)-3-(benzyloxy)tetradecanoylamino]-2- deoxy-α-D-glucopyranoside (Compound 21*)

NaB³H₄ (590 mL, 26.4 mmol/mL, 240 GBq mmol⁻¹) was added at 0°C to a solution of the compound 20 (150 mg, 62.4 mmol) in 2-propanol/methanol/CH₂Cl₂ (5:1:1, 3.5 mL) and stirred for 30 minutes. The reaction was stopped by adding a saturated aqueous solution of ammonium chloride followed by extracting with ethyl acetate. The extract was dried over Na₂SO₄ and concentrated *in vacuo* to obtain a colorless syrup of the compound 21 * (144 mg, quantitatively). The product was used for the next reaction without purification.

### (1-3) Synthesis of 2-(1,5-dihydro-3-oxo-3λ⁵-3H-2,4,3- benzodioxaphosphepin-3-yloxy) -[2-³H₁]ethyl 4-O-benzyl-6-O-[6-O-benzyl-2-deoxy-4-O-(1,5-dihydro-3-oxo-3λ⁵-3H-2,4,3-benzodioxaphosphepin-3-yl)-2-[(R)-3-(dodecanoyloxy)tetradecanoylamino]-3-O-[(R)-3-(tetradecanoyloxy)tetradecanoyl]-β-D-glucopyranosyl]-3-O-[(R)-3-(benzyloxy) tetradecanoyl]-2-[(R)-3-(benzyloxy)tetradecanoylamino]-2-deoxy-α-D-glucopyranoside (Compound 22*)

N,N-Diethyl-1,5-dihydro-3*H*-2,4,3-benzodioxaphosphepin-3-amine (89 mg, 0.37 mmol) and tetrazole (25 mg, 0.32 mmol) were added at 0°C to a solution of the compound 21* (144 mg, 59.9 mmol) in CH₂Cl₂ (14 mL). After the reaction mixture was stirred at room temperature for 30 minutes, it was cooled at -78°C. mCPBA (70%, 81 mg, 0.37 mmol) was added thereto followed by stirring for 45 minutes. The reaction was stopped by adding a saturated Na₂S₂O₃ followed by extracting with CHCl₃. The organic layer was washed with a saturated aqueous solution of NaHCO₃ and a saturated saline solution and dried over Na₂SO₄. After it was concentrated *in vacuo*, the residue was purified by a silica gel column chromatography [3.0 g, toluene/ethyl acetate/hexafluoro-2-propanol (HFIP) = 100:50:1] to obtain a colorless syrup of the compound 22* (134 mg, yield: 87%). The resulting compound was identical with an authentic sample of non-labeled 22 on the TLC.
[α]_{D}²⁵ = +17.8 (*c* 1.00, CHCl₃); FAB-MS (positive) *m*/*z* 2428 [(M+Na)⁺]ₒ Found: C, 69.96; H, 9.21; N, 1.14%. Calcd for C₁₄₀H₂₁₈N₂O₂₆P₂: C, 69.85; H, 9.13; N, 1.16%.

### (1-4) Synthesis of 4-(phosphonoxy)[2-³H₁]ethyl 2-deoxy-6-O-[2-deoxy-2-[(R)-3-(dodecanoyloxy)tetradecanoylamino]-4-O-phosphono-3-O-[(R)-3-(tetradecanoyloxy) tetradecanoyl]-β-D-glucopyranosyl]-3-O-[(R)-3-hydroxytetradecanoyl]-2-[(R)-3-hydroxytetradecanoylamino]-α-D-glucopyranoside ([³H] PE 506: 4*)

Pd-black (130 mg) was added to a solution of the compound 22* (57 mg, 22 mmol) in THF (8 mL) and a catalytic reduction was carried under pressure (7 kg cm⁻²) at room temperature for 2 hours. Triethylamine was added to neutralize and the Pd-black was removed by filtration. The residue obtained by a vacuum concentration was purified by a liquid-liquid partition chromatography (Sephadex LH-20: 20 g, CHCl₃/methanol/water/2-propanol = 9:9:9:1) (organic layer was used as a fixed phase while aqueous layer was used as a mobile phase) to obtain [³H] PE 506: 4*) as white powder (25 mg, 841 MBq, 62 GBq mmol⁻¹, yield: 63%). The resulting compound was identical with an authentic sample of non-labeled 4 on a chromatography.
FAB-MS (negative) *m*/*z* 1840 [(M-H)⁻]; ¹H NMR (600MHz,CD₃OD/CDCl₃=1:1) δ =5.23 (m,1H), 5.19 (t,*J* =8.2 Hz,1H), 5.17 (m,1H), 5.14 (t,*J* =8.2 Hz,1H), 4.82 (d,*J* =3.0 Hz,1H), 4.56 (d,*J* =7.4 Hz,1H), 4.23 (q,*J* =8.0Hz,1H), 4.18 (dd,*J* =8.9, 3.0Hz,1H), 4.08-3.93 (m,5H), 3.92-3.82, (m,4H), 3.80 (dd,*J* =10.3, 4.5Hz,1H), 3.74 (d,*J* =10. 6 Hz,1H), 3.63 (m,1H), 3.56 (t,*J* =8.1Hz,1H),3.37 (m,1H), 2.72 (dd,*J* =14.0, 6.6Hz,1H), 2.64 (dd,*J* =14.0, 4.5Hz,1H), 2.52-2.46 (m,2H), 2.44-2.36 (m,2H), 2.36-2.27 (m,6H), 1.66-1.39 (m,12H),1.38-1.20 (m,108H), 0.89 (t,*J* =5.6Hz,18H).

### (2) Synthesis of 2-(phosphonoxy)[2-³H₁]ethyl 2-deoxy-6-O-[2-deoxy-2-[(R)-3-hydroxytetradecanoylamino]-4-O-phosphono-3-O-[(R)-3-hydroxytetradecanoyl]-β-D-gl ucopyranosyl]-3-O-[(R)-3-hydroxytetradecanoyl]-2-(R)-3-hydroxytetradecanoylamino]-α-D-glucopyranoside ([³H] PE 406: 5*)

### (2-1) Synthesis of formylmethyl 4-O-benzyl-6-O-[6-O- benzyl-2-deoxy-4-O-(1,5-dihydro-3-oxo-3λ⁵-3H-2,4,3-benzodioxaphosphepin-3-yl)-2-[(R)-3-(benzyloxy) tetradecanoylamino]-3-O-[(R)-3-(benzyloxy)tetradecanoyl]-β-D-glucopyranosyl]-3-O-[(R)-3-(benzyloxy)tetradecanoyl]-2-[(R)-3-(benzyloxy)tetradecanoylamino]-2-deoxy-α-D-glucopyranoside (Compound 24)

An aqueous solution (2.5%, 230 mL, 20 mmol) of 4-methylmorpholine N-oxide (NMO) (40 mg, 0.45 mmol) and osmium tetraoxide (OsO₄) was added to a solution of the compound 23 (230 mg, 0.15 mmol) in THF/tert-butanol/water (10:10:1, 9 ml) with vigorous stirring. After 6 hours, a saturated aqueous solution of Na₂S₂O₃ (50 mL) was added followed by extracting with ethyl acetate (50 mL). The organic layer was washed with an aqueous solution of Na₂S₂O₃ (2 × 50 mL) and a saturated saline solution (20 mL), dried over Na₂SO₄ and concentrated *in vacuo* to obtain a diol as a crude product (458 mg). The diol was dissolved in anhydrous benzene (10 mL) and lead tetraacetate (Pb(OAc)₄) (purity: 90%, 68 mg, 140 mmol) was added thereto followed by stirring for 30 minutes. The reaction mixture was charged to a silica gel column (3 g) and eluted with ethyl acetate. The eluate was concentrated *in vacuo* and the residue was purified by a medium-pressure silica gel chromatography (6 g, toluene/ethyl acetate = 4:1) to obtain the compound 24 as a colorless syrup (190 mg, yield: 83%).
FAB-MS (positive) *m*/*z* 2032 [(M+Na)⁺]; ¹H NMR (500MHz,CDCl₃) δ =9.30 (s, 1H, CHO).

### (2-2) Synthesis of 2-hydroxy-[2-³H₁]ethyl 4-O-benzyl-6-O-[6-O-benzyl-2-deoxy-4-O-(1,5-dihydro-3-oxo-3λ⁵-3H-2,4,3-benzodioxaphosphepin-3-yl)-2-[(R)-3-(benzyloxy) tetradecanoylamino]-3-O-[(R)-3-(benzyloxy)tetradecanoyl]-β-D-glucopyranosyl]-3-O-[(R)-3-(benzyloxy)tetradecanoyl]-2-[(R)-3-(benzyloxy)tetradecanoylamino]-2-deoxy-α-D-glucopyranoside (Compound 25*)

NaB³H₄ (474 mL, 26.3 mmol/mL, 240 GBq mmol⁻¹) was added at 0°C to a solution of the compound 24 (100 mg, 49.8 mmol) in 2-propanol/methanol (5:1, 1.5 mL) and stirred for 30 minutes. The reaction was stopped by adding a 2M aqueous solution of HCl and a saturated saline solution followed by extracting with ethyl acetate. The extract was dried over Na₂SO₄ and concentrated *in vacuo* to obtain a colorless syrup of the compound 25* (100 mg, quantitatively). The product was used for the next reaction without purification.

### (2-3) Synthesis of 2-(1,5-dihydro-3-oxo-3λ⁵-3H-2,4,3- benzodioxaphosphepin-3-yloxy) -[2-³H₁]ethyl 4-O-benzyl-6-O-[6-O-benzyl-2-deoxy-4-O-(1,5-dihydro-3-oxo-3λ⁵-3H-2,4,3-benzodioxaphosphepin-3-yl)-2-[(R)-3-(benzyloxy)tetradecanoylamino]-3-O-[(R)-3-(benzyloxy)tetradecanoyl]-β-D-glucopyranosyl]-3-O-[(R)-3-(benzyloxy)tetradecanoyl ]-2-[(R)-3-(benzyloxy)tetradecanoylamino]-2-deoxy-α-D-glucopyranoside (Compound 26*)

N,N-Diethyl-1,5-dihydro-3H-2,4,3-benzodioxaphosphepin-3-amine (70 mg, 0.29 mmol) and tetrazole (20 mg, 0.25 mmol) were added at 0°C to a solution of the compound 25* (100 mg, 49.8 mmol) in CH₂Cl₂ (14 mL). After the reaction mixture was stirred at room temperature for 30 minutes, it was cooled at -20°C. mCPBA (70%, 65 mg, 0.29 mmol) was added thereto followed by stirring for 45 minutes. The reaction was stopped by adding a saturated Na₂S₂O₃ followed by extracting with CHCl₃. The organic layer was washed with a saturated aqueous solution of NaHCO₃ and a saturated saline solution and dried over Na₂SO₄. After it was concentrated *in vacuo*, the residue was purified by a silica gel column chromatography [2.3 g, toluene/ethyl acetate/hexafluoro-2-propanol (HFIP) = 100:50:1] to obtain a colorless syrup of the compound 26* (63.9 mg, yield: 59%). The resulting compound was identical with an authentic sample of non-labeled 26 on the chromatography.
[α]D²⁵ = +16.9 (c 1.00, CHCl₃); FAB-MS (positive) *m*/*z* 2428 [(M+Na)⁺].
Found: C, 69.98; H, 8.40; N, 1.33%. Calcd for C₁₂₈H₁₈₂N₂O₂₄P₂: C, 70.05; H, 8.36; N, 1.28%.

### (2-4) Synthesis of 2-(phosphonoxy)[2-³H₁]ethyl 2-deoxy-6-O-[2-deoxy-2[(R)-3-hydroxytetradecanoylamino]-4-O-phosphono-3-O-[(R)-3-hydroxytetradecanoyl]-β-D-glucopyranosyl]-3-O-[(R)-3-hydroxytetradecanoyl]-2-[(R)-3-hydroxytetradecanoylamino]-α-D-glucopyranoside ([³H] PE 406: 5*)

Pd-black (120 mg) was added to a solution of the compound 26* (17 mg, 7.8 mmol) in THF (6 mL) and a catalytic reduction was carried under pressure (7 kg cm⁻²) at room temperature for 2 hours. Triethylamine was added to neutralize and the Pd-black was removed by filtration. The residue obtained by a vacuum concentration was purified by a liquid-liquid partition chromatography (Sephadex LH-20: 20 g, CHCl₃/methanol/water/2-propanol = 8:8:9:1) (organic layer was used as a fixed phase while aqueous layer was used as a mobile phase) to obtain [³H] PE 406: 5*) as white powder (7.5 mg, 331 MBq, 64 GBq mmol⁻¹, yield: 66%). The resulting compound was identical with an authentic sample of non-labeled 5 on a chromatography.
FAB-MS (negative) *m*/*z* 1448 [(M-H)⁻]; ¹H NMR (500MHz, SDS-d₂₅-D₂O) *d* = 5.18(dd,*J* =9.4, 7.5Hz,1H), 5.17 (dd,*J* =9.8, 9.6Hz,1H), 4.87 (d,*J* = 3.8 Hz,1H), 4.67 (d,*J* =7.0Hz,1H), 4.12 (dd,*J* =9.8, 3.8 Hz,1H), 4.11 (dd,*J* =15.5,1.6Hz,1H), 4.11 (q,*J* =7.5,1H), 4.02 (dd,*J* =9.4, 7.0Hz,1H), 3.99 (m,1H), 3.96 (m,1H), 3.91 (m,3H), 3.87 (ddd,*J* =9.6, 1.6, 2.9Hz,1H), 3.82 (dd,*J* =9.8, 8.6Hz,1H), 3.82 (dd,*J* =15.5, 2.9Hz,1H), 3.82 (m,1H), 3.81 (m,3H), 3.71 (m,1H), 3.59 (m,1H), 2.56-2.23 (m,8H), 1.54-1.11 (m,80H), 0.86 (m,12H).

### Example 2 Preparation of molecular tweezers library (peptide library)

An encode molecular tweezers library was prepared by a split-mix synthetic method. As a solid carrier, 15 g of Tenta Gel S-NH₂ (Rapp Polymere; particle size: 90 µm; 0.29 mmol/g of NH₂) were used. A peptide chain was elongated using a standard Fmoc/Boc (fluorenylmethoxycarbonyl/butyloxycarbonyl) peptide solid-phase synthetic method. As a peptide condensation method, HOBt/DIC (hydroxybenzotriazole/diisopropyl carbodiimide) was used. The end point of the peptide condensation reaction was confirmed by disappearance of blue color by a bromophenol blue test. Catechol Tag was introduced by an amide bond formation reaction in about 1% of the amino groups by an HOBt/DIC method via a carboxyl group. When chenodeoxycholic acid (L1) was used as a core structure, deoxycholic acid was firstly introduced into a solid phase carrier and 6 equivalents of Fmoc-Gly-F, 6 equivalents of triethylamine and 0.06 equivalent of dimethylaminopyridine were made to act with two hydroxyl groups in DMF whereby Fmoc-Gly was bound. Lysine (L2) and trilysine (L3) cores were prepared by a common peptide solid phase synthetic method. In preparing a library, 15 kinds (Gly with D and L-Val, Phe, Ser, Gln, Glu, Lys and Pro) of amino acids were used. A split coupling cycle in four stages was carried out to construct a library containing 3 × 15⁴ = 151,875 kinds of peptides. Protective group on the library was removed using 20% piperidine/DMF and 95% TFA/2.5% water/2.5% thioanisole and stored at 4°C.

### Example 3 Binding assay of the molecular tweezers library (peptide library) to radioisotope-labeled lipid A analog

### (1) Binding assay to [³H] PE 506

[³H] PE 506 (62 GBq/mmol, 1.4 mg, 47 MBq) was dissolved in water (2 ml). A molecular tweezers library (15.7 mg, about 19,000 beads) was suspended in water (200 µl) and a [³H] PE 506 solution (100 µl, 2.4 MBq) was added. The suspension of [³H] PE 506 (130 µM) and resin was slowly shaken at 4°C for 41 hours. An operation where the resin was precipitated by centrifugation and the supernatant liquid was removed was repeated three times. Glass plate was treated with an NaCIO solution, well washed with water, taken out after dipping in 0.5 gelatin solution and dried with air to give a glass plate on which gelatin was coated. The above resin was placed thereon and dried with air. This was covered with a Kodak autoradiography emulsion which was previously melted by warming and, after confirming that it was well solidified, it was allowed to stand under shading at -78°C for 12 days. After the glass plate was dipped in a developing liquid for 10 minutes, it was washed with water. After being dipped in a fixing solution for 15 minutes, it was washed with water. Surroundings of the resin having radioactivity became black by adhesion of silver to gelatin. Using it as an index, the radioactive resin was taken out and Tag bound to the resin was cut from the resin, detected by an electron capture gas chromatography (ECGC) and decoded to find molecular tweezers binding to 19 kinds of [³H] PE 506.

### (2) Binding assay to [³H] PE 406

[³H] PE 406 (64 GBq/mmol, 0.6 mg, 26 MBq) was dissolved in water (1 ml). A molecular tweezers library (24.2 mg, about 29,000 beads) was suspended in water (200 µl) and a [³H] PE 406 solution (100 µl, 2.6 MBq) was added. The suspension of [³H] PE 406 (130 µM) and resin was slowly shaken at 4°C for 48 hours. The resin was precipitated by centrifugation and the supernatant liquid was removed. An operation where 1 µl of water is added to the resin, the resulting mixture is shaken, the resin is precipitated by centrifugation and the supernatant liquid is removed was repeated three times.

Resin was placed on a gelatin-coated glass plate and dried with air. This was covered with a Kodak autoradiography emulsion which was previously melted by warming and, after confirming that it was well solidified, it was allowed to stand under shading at -78°C for 7 days. The radioactive resin was taken out and subjected to an ECGC analysis. A molecular tweezers binding to 32 kinds of [³H] PE 406 was found.

### Example 4 Re-synthesis of molecular tweezers

As a solid phase carrier, Tenta Gel S-NH₂ (Rapp Polymere; particle size: 90 µm; 0.29 mmol/g of NH₂) or TSK gel AF-Amino TOYOPEARL 650 S (Tosoh; 0.1 mmol/ml) was used. Peptide chain was elongated using a standard Fmoc/Boc (fluorenylmethoxycarbonyl/butyoxycarbonyl) peptide solid phase synthetic method. As a peptide condensation method, an HOBt/DIC (hydroxybenzotriazole/diisopropylcarbodiimide) was used. The end point of the peptide condensation reaction was confirmed by disappearance of blue color by a bromophenol blue test.

As a representative example, a synthetic example for a molecular tweezers 6-Toyopearl [(k-s-k-S)₄L3-Toyopearl] will be shown.

### (1) Introduction of Nα,Nε-di-tert-butoxycarbonyl-L-lysine (Boc-L-Lys(Boc)-OH)

A 10% diisopropylethylamine (DIEA)/DMF (4.0 ml) was added to TSK gel AF-Amino TOYOPEARL 650 S (0.1 mmol/ml)(1ml, 0.1mmol), shaken for 30 seconds and filtered. The resin was washed with DMF (4.0 ml) twice and a DMF solution (3 ml) of Boc-L-Lys(Boc)-OH (104 mg, 0.300 mmol), a DMF solution (1 ml) of diisopropylcarbodiimide (DIC) (47.0 µl, 0.300 mmol) and a DMF solution (1 ml) of 1-hydroxybenzotriazole (HOBt) (40.5 mg, 0.300 mmol) were added thereto followed by stirring at room temperature for 2 hours. After filtration, the resin was washed with DMF (4.0 ml) four times. A peptide condensation reaction using Boc-L-Lys(Boc)-OH was repeated once again. A solution (2 ml) of acetic anhydride-DMF (1:1) and a solution (2 ml) of triethylamine-DMF (1:1) were added followed by shaking at room temperature for 15 minutes whereby slightly-remaining unreacted amino groups were completely blocked. After filtration, the resin was washed with DMF (4 ml) four times.

### (2) Removal of Boc group

A 25% TFA/DMF (4.0 ml) was added followed by shaking at room temperature for 30 minutes. After filtration, the resin was washed with DMF (4.0 ml) four times.

### (3) Condensation of Nα-fluorenylmethoxycarbonyl-Nε- tert-butoxycarbonyl-L-lysine (Fmoc-L-Lys(Boc)-OH)

A DMF solution (3 ml) of Fmoc-L-Lys(Boc)-OH (281 mg, 0.600 mmol), a DMF solution (1 ml) of DIC (94.0 µl, 0.600 mmol) and a DMF solution of HOBt (81.1 mg, 0.600 mmol) were added followed by shaking at room temperature for 2 hours. After filtration, the resin was washed with DMF (4.0 ml) for four times. A peptide condensation reaction using Fmoc-L-Lys(Boc)-OH was repeated once again. Unreacted amino groups were similarly blocked using acetic anhydride.

### (4) Removal of Fmoc group

A 20% piperidine/DMF (4 ml) was added followed by shaking at room temperature for 30 minutes whereby Fmoc group was removed. After filtration, the resin was washed with DMF (4.0 ml) three times and with methanol (4.0 ml) twice. An operation of removal of the Fmoc group was repeated twice.

### (5) Removal of Boc group

Boc group was removed by the same manner as in the above (2).

### (6) Condensation of Nα-fluorenylmethoxycarbonyl-o-tert-butyl-L-serine (Fmoc-L-Ser(tBu)-OH)

A DMF solution (6 ml) of Fmoc-L-Ser(tBu)-OH (460 mg, 1.20 mmol), a DMF solution (2 ml) of DIC (188 µl, 1.20 mmol) and a DMF solution (2 ml) of HOBt (162 mg, 1.20 mmol) were added followed by shaking at room temperature for 2 hours. After filtration, the resin was washed with DMF (4.0 ml) four times.

### (7) Removal of Fmoc group

Fmoc group was removed by the same manner as in the above (4).

### (8) Condensation of Fmoc-D-Lys(Boc)-OH

A DMF solution (6 ml) of Fmoc-D-Lys(Boc)-OH (562 mg, 1.20 mmol), a DMF solution (2 ml) of DIC (188 µl, 1.20 mmol) and a DMF solution (2 ml) of HOBt (162 mg, 1.20 mmol) were added followed by shaking at room temperature for 2 hours. After filtration, the resin was washed with DMF (4.0 ml).

### (9) Removal of Fmoc group

Fmoc group was removed by the same manner as in the above (4).

### (10) Condensation of Fmoc-D-Lys(Boc)-OH

A DMF solution (6 ml) of Fmoc-D-Lys(Boc)-OH (562 mg, 1.20 mmol), a DMF solution (2 ml) of DIC (188 µl, 1.20 mmol) and a DMF solution (2 ml) of HOBt (162 mg, 1.20 mmol) were added followed by shaking at room temperature for 2 hours. After filtration, the resin was washed with DMF (4.0 ml).

### (11) Removal of Fmoc group

Fmoc group was removed by the same manner as in the above (4).

### (12) Removal of Boc group and tBu group

The protective groups at side chain were removed by the same manner as in the removal operation of Boc group in the above (2).

Thus, the operations of the above (1) to (12) were successively carried out whereupon molecular tweezers [(k-s-k-S)₄L3-Toyopearl] was prepared.

Other molecular tweezers resin was also synthesized by the same manner as above.

### Example 5 Binding assay of the molecular tweezers 6-Tenta Gel to 17-Tenta Gel, to PE 506, 506, PE 406, 406, ReLPS and Escherichia coli lipopolysaccharide (LPS)

PE 506 (0.06 mg) was dissolved in water (0.80 ml). The molecular tweezers 6 to 17 Tenta Gel (about 20 mg) were suspended in water (100 µl) and a PE 506 solution (25 µl) was added thereto. The suspension was slowly shaken overnight at room temperature. The resin was precipitated by centrifugation and the supernatant liquid was used for a TLC analysis and a Limulus activity test.

Binding assays were similarly carried out using 506 (0.56 mg), PE 406 (0.7 mg), 406 (0.7 mg), PeLPS (0.85 mg) and LPS (1 mg) and the resulting supernatant liquids were used for the measurement of Limulus activity (in the case of 506, it was used for a TLC analysis as well). The result of the TLC analysis is as shown in Table 6.

### Example 6 Binding assays of molecular tweezers 6-Toyopearl, 11-Toyopearl, 12-Toyopearl, 16-Toyopearl, and 17-Toyopearl; to 506, ReLPS, and Escherichia coli lipopolysaccharide (LPS)

A solution where 506 (0.56 mg) was dissolved in water (0.80 ml) was further diluted to an extent of 3-fold. Each Toyopearl (about 20 mg) in the molecular tweezers was suspended in water (100 µl) and a 506 solution (25 µl) was added. The suspension was slowly shaken overnight at room temperature. The resin was precipitated by centrifugation and the supernatant liquid was used for the measurement of Limulus activity.

In the case of ReLPS (0.85 mg) and *Escherichia coli* lipopolysaccharide (LPS) (1 mg), the above solution was similarly diluted to an extent of 3-fold, and then binding assays were similarly carried out. The resulting supernatant liquid was used for the measurement of Limulus activity.

### Example 7 Measurement of Limulus activity

Measurement of Limulus activity was carried out using ENDOSPECY R (manufactured by Seikagaku Kogyo).
(1) Each of the samples (PE 506, 506, PE 406, 406, ReLPS and *Escherichia coli* lipopolysaccharide (LPS)) was dissolved in water to prepare a solution of 1 mg/ml.
(2) The sample was diluted to an appropriate concentration on a 96-well microplate using distilled water for injection (manufactured by Otsuka Pharmaceutical).
(3) Each 20 µl of the sample solution were placed in a microplate and 30 µl of an ENDOSPECY R ES-50M set LAL reagent were added under cooling in an ice water bath, followed by warming at 37°C for 20 minutes.
(4) Each 75 ml of the following diazotizing reagents A, B, C were added successively and absorbance at 540 nm wavelength was measured. The absorbance when each sample concentration was 10⁻⁶ mg/ml was used as a standard.

### (Diazotizing reagents)

A: a solution where 40 mg of sodium nitrite was dissolved in 4 ml of concentrated hydrochloric acid and 96 ml of water

B: a solution where 300 mg of ammonium sulfamate was dissolved in 100 ml of water

C: a solution where 70 mg of N-naphthylethylenediamine dihydrochloride was dissolved in 100 ml of water

Each of the samples obtained in the binding assays was also diluted in the entirely same manner, subjected to a Limulus activity test, and the absorbance was measured. By comparing with the standard absorbance of each of the above samples, adsorbing ability of each resin was estimated. The result is shown in Fig. 1 and Fig. 2.

### Industrial Applicability

The invention relates to methods for screening and preparation of an endotoxin adsorbent and to an adsorbent having a specific structure which was found by that methods. The advantages are that it provides a novel endotoxin adsorbent where synthesis is easy, various analogous structures can be easily prepared and can be used for an adsorbent of various endotoxins. In addition, as compared with the existing adsorbents, the adsorbent of the invention has a big advantage of using a natural product (natural amino acid).

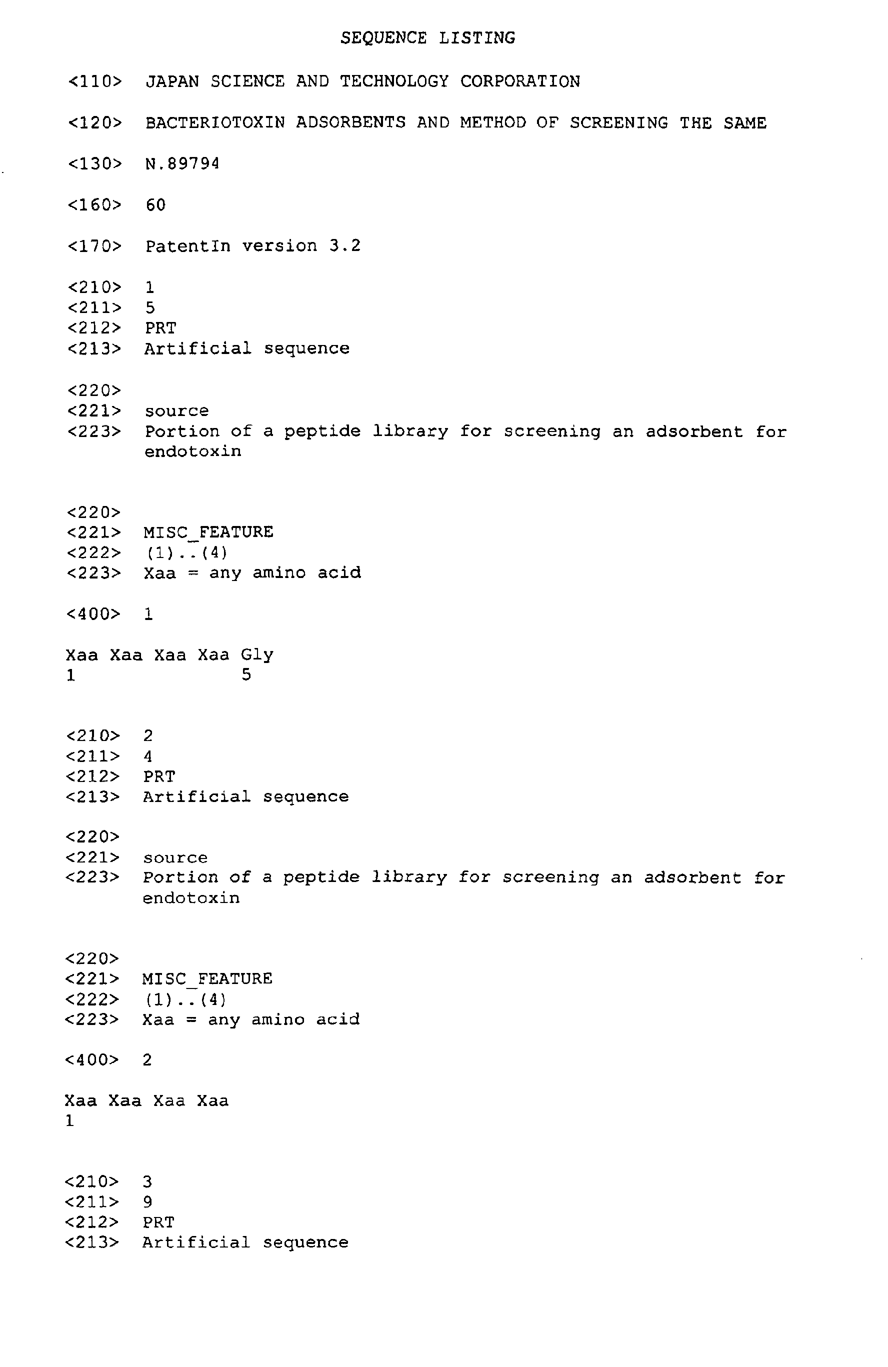

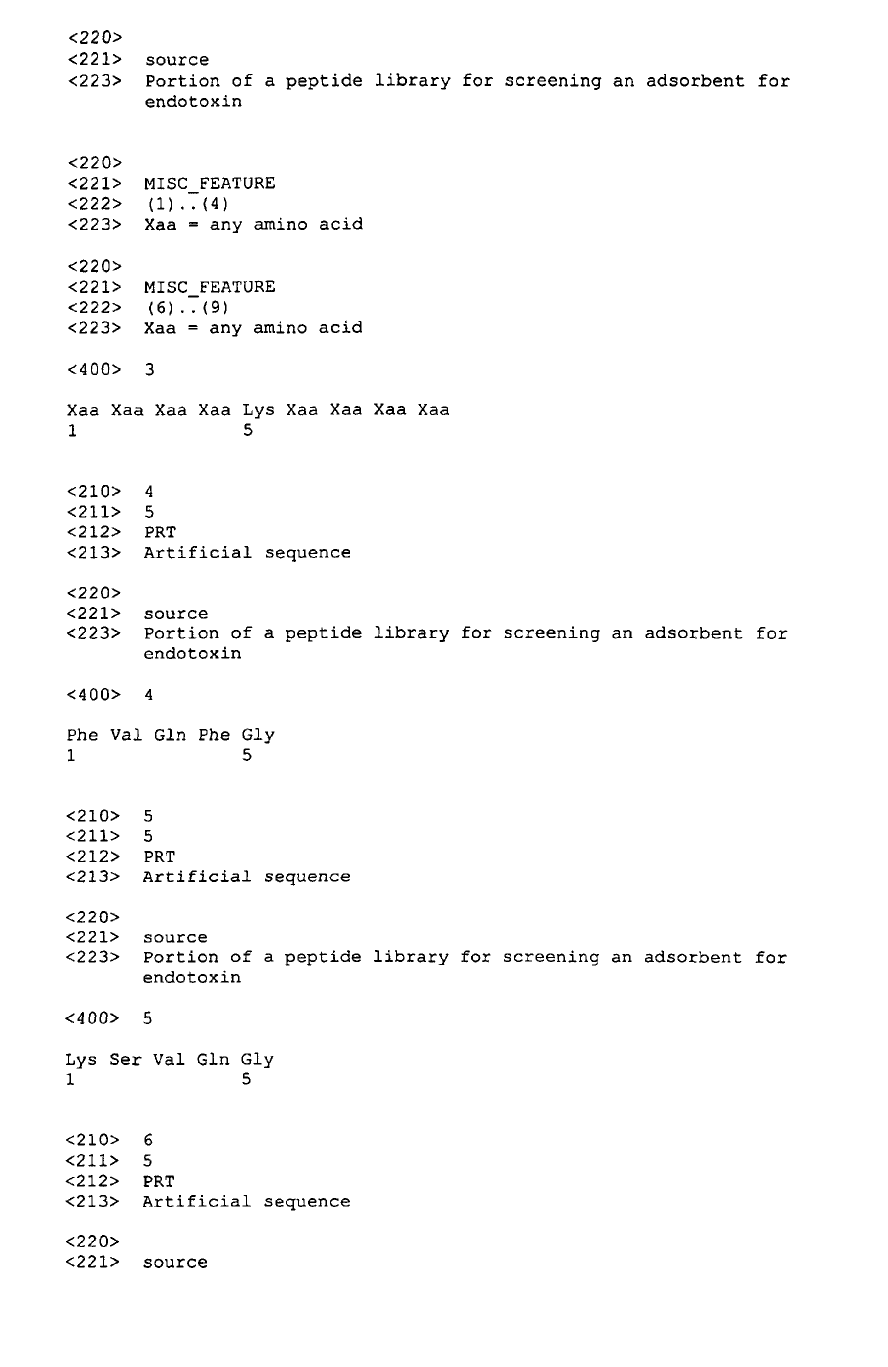

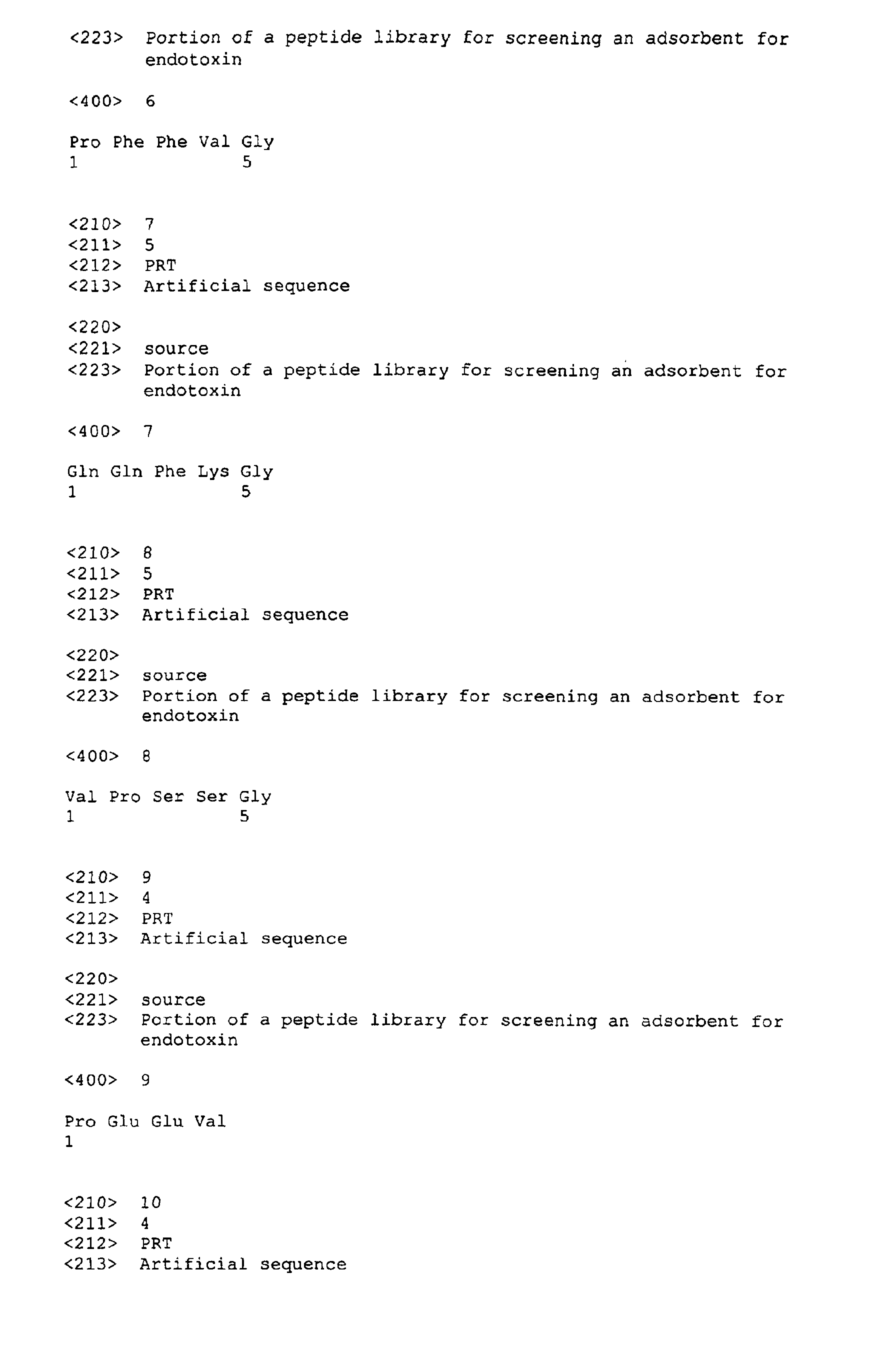

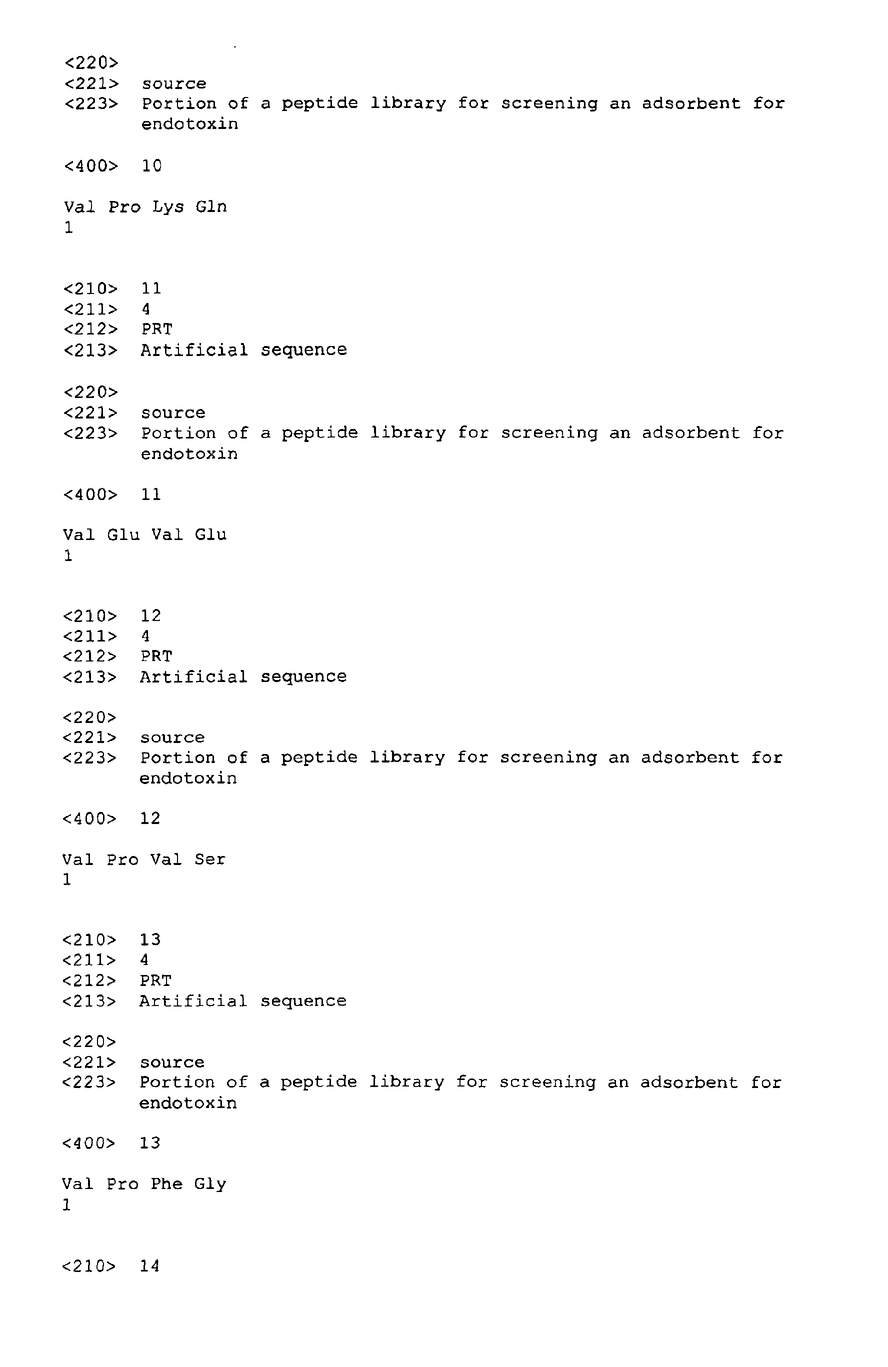

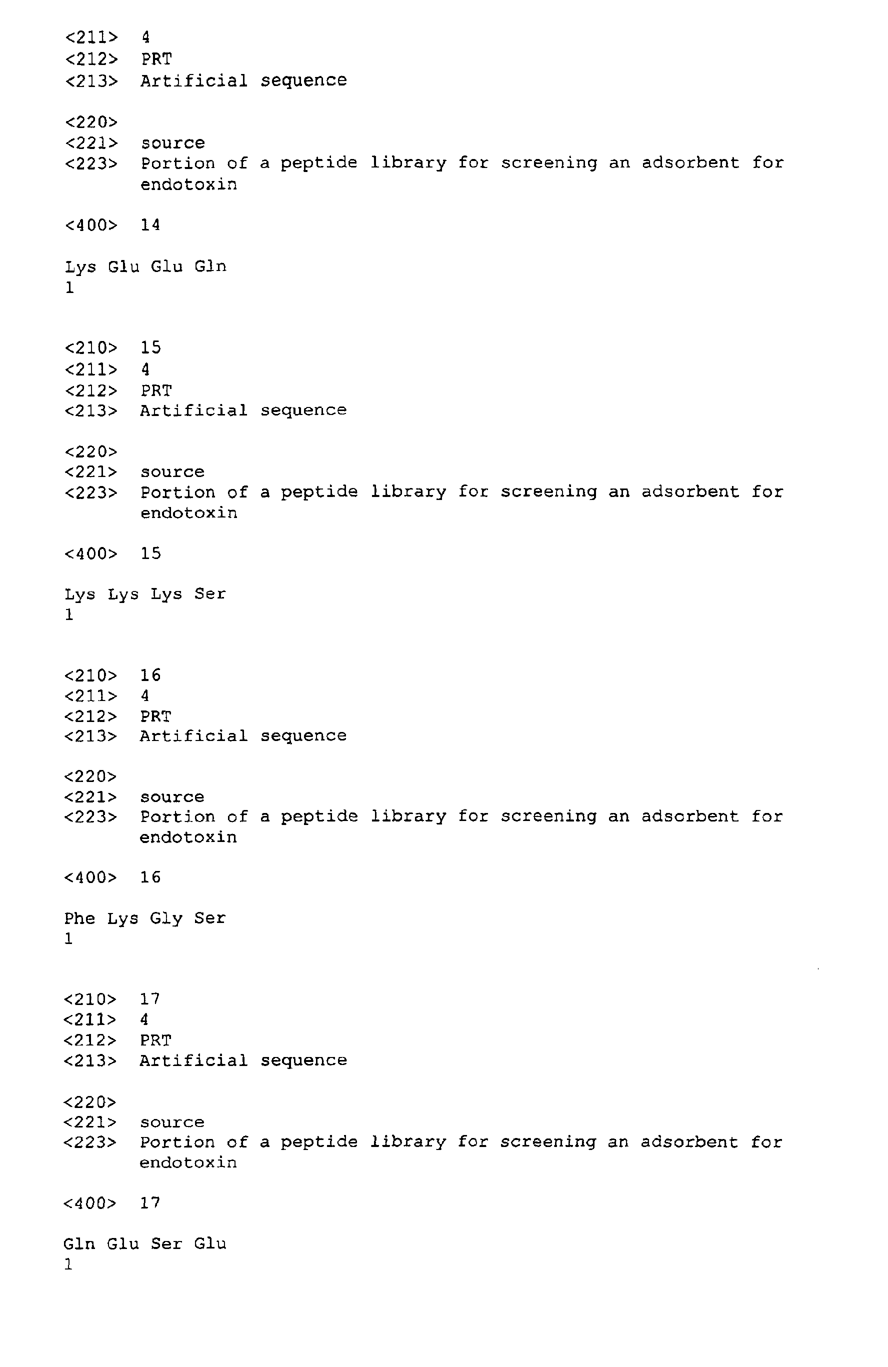

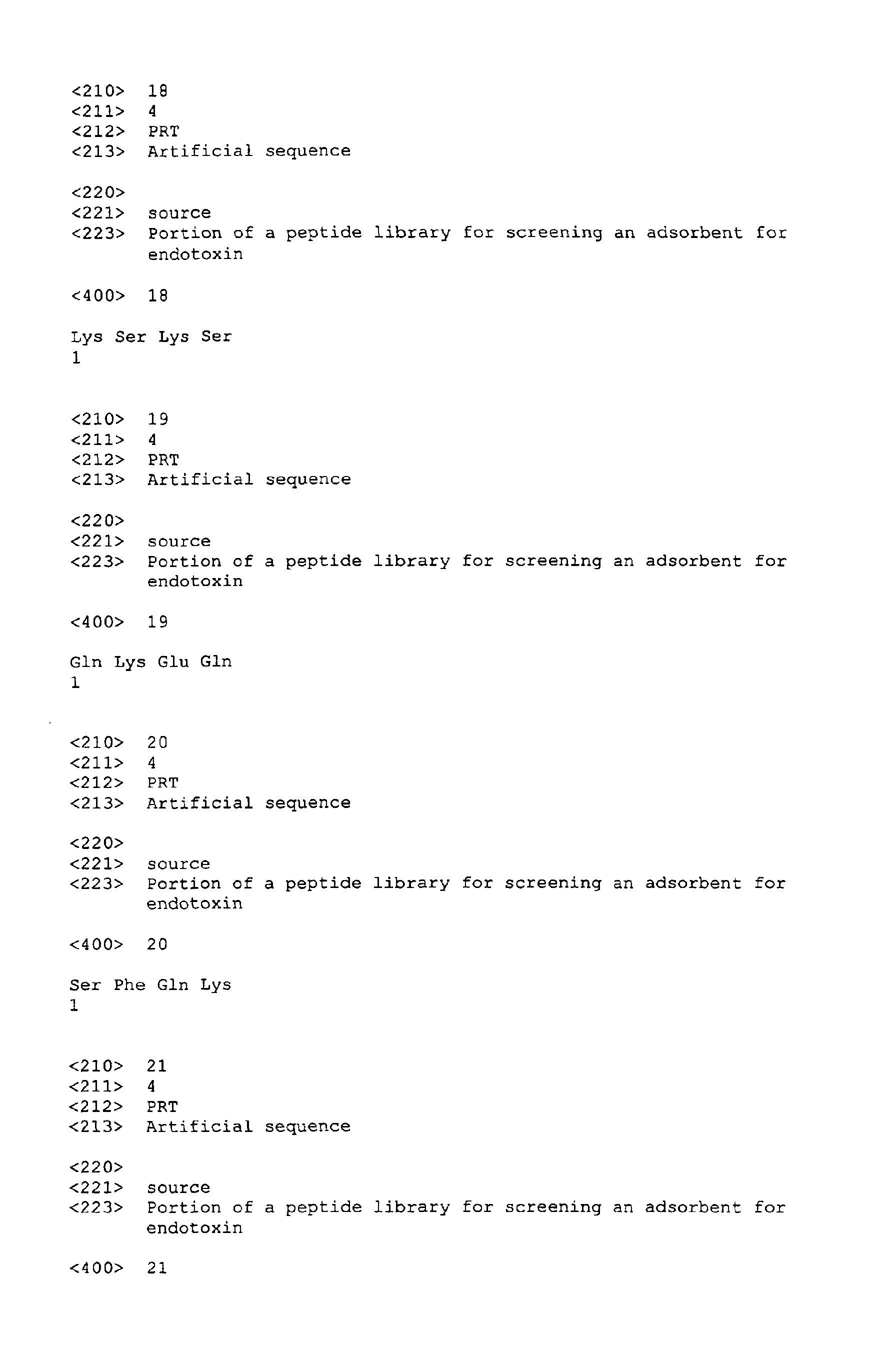

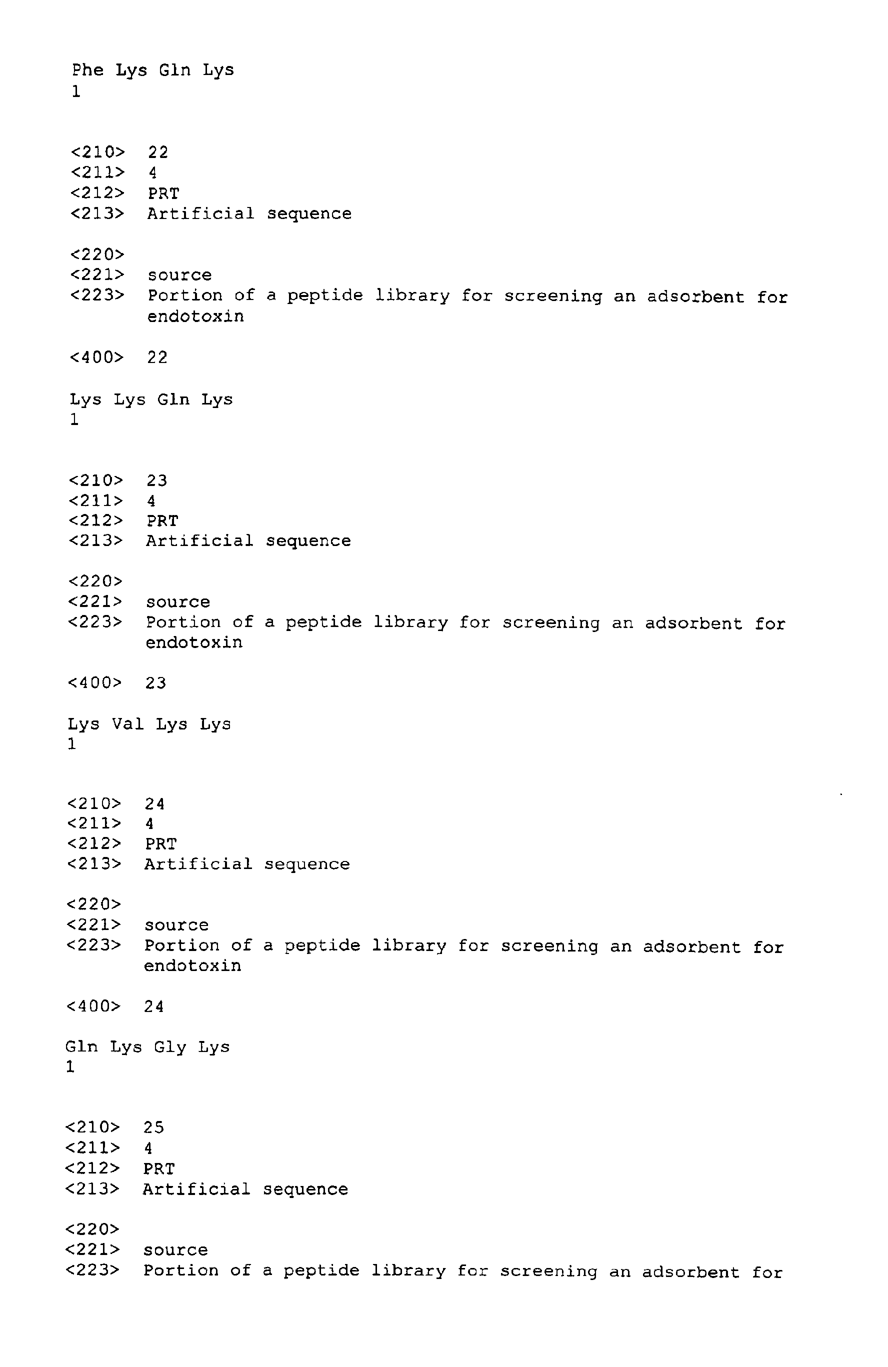

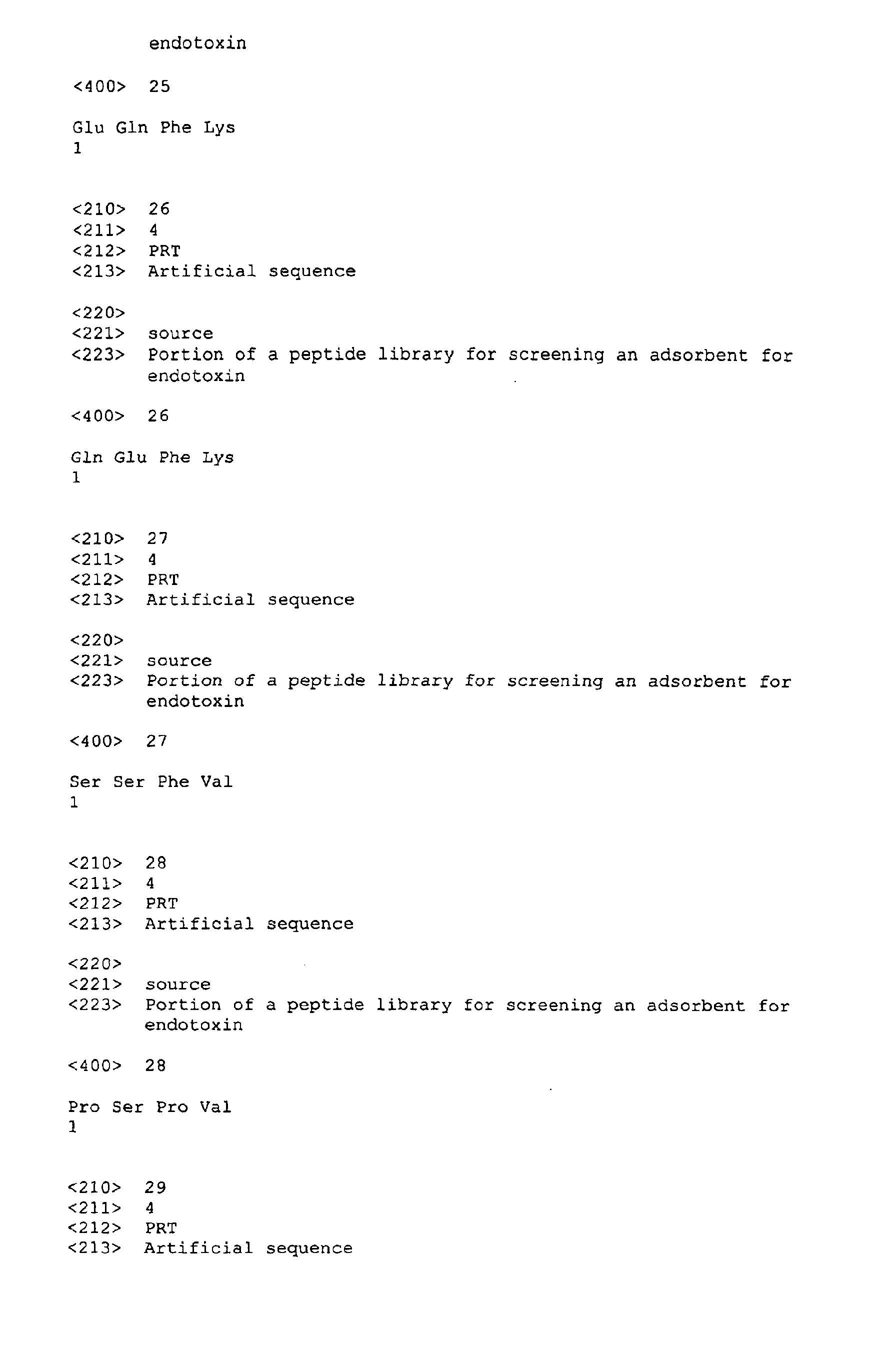

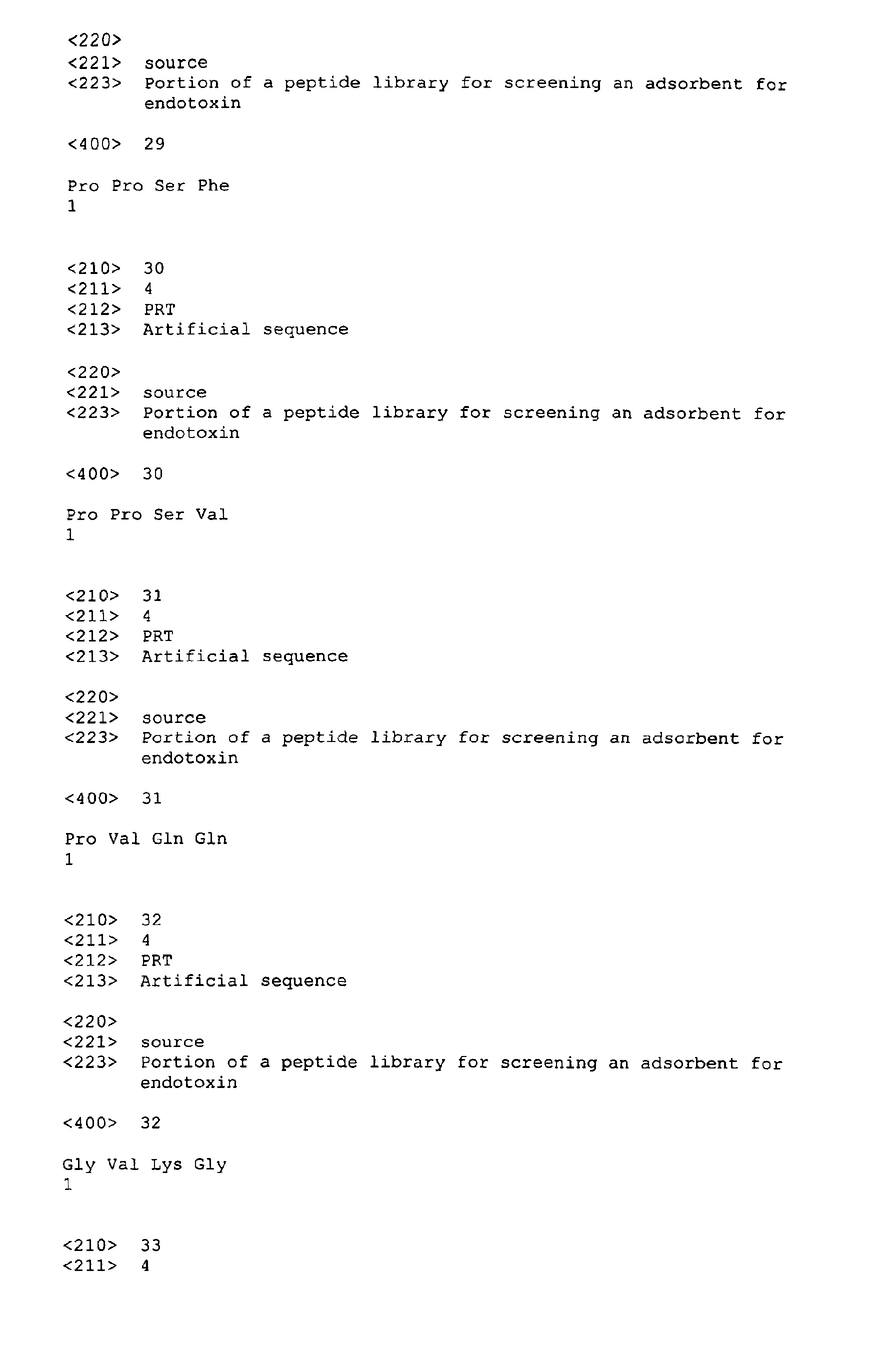

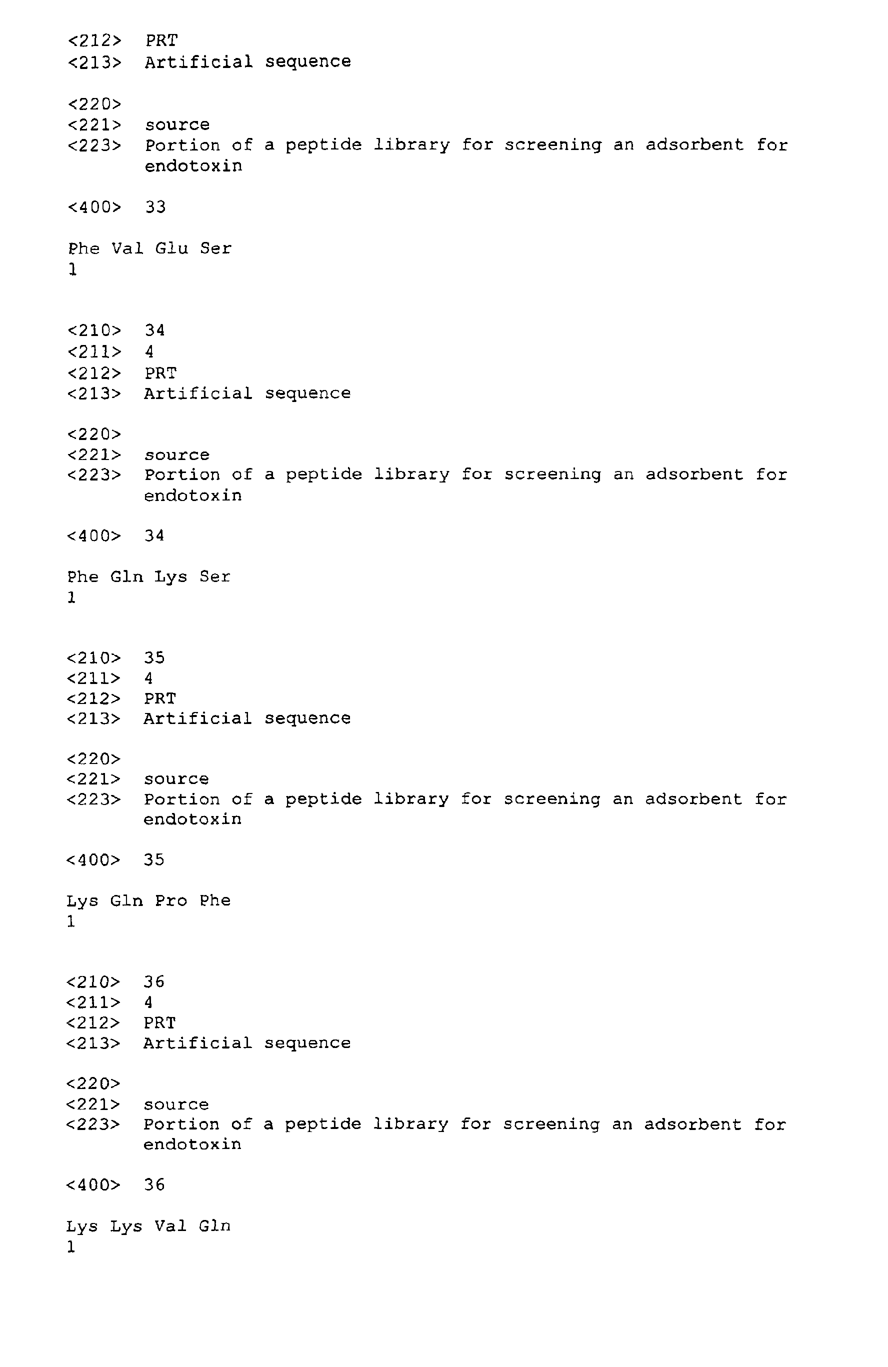

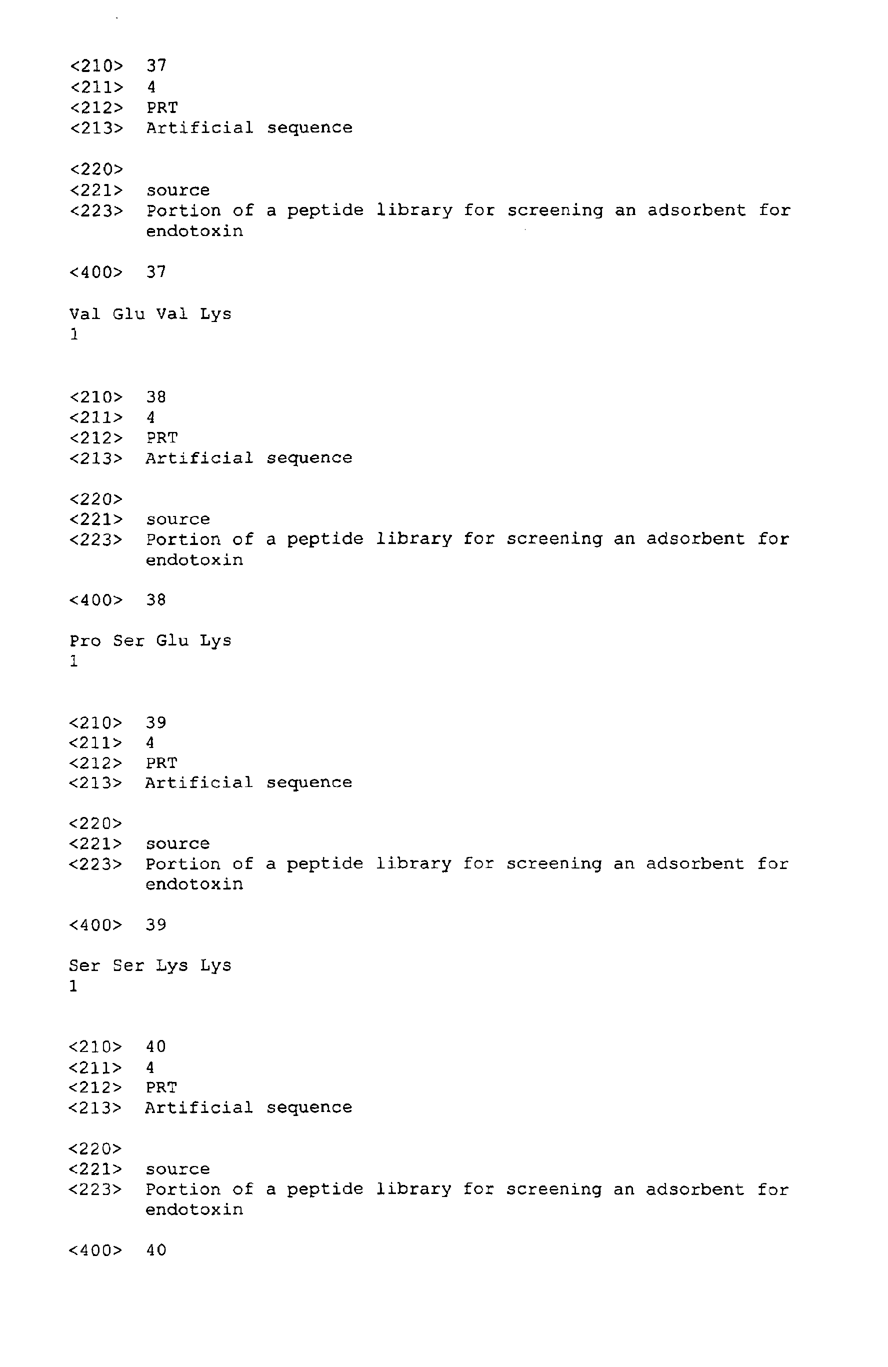

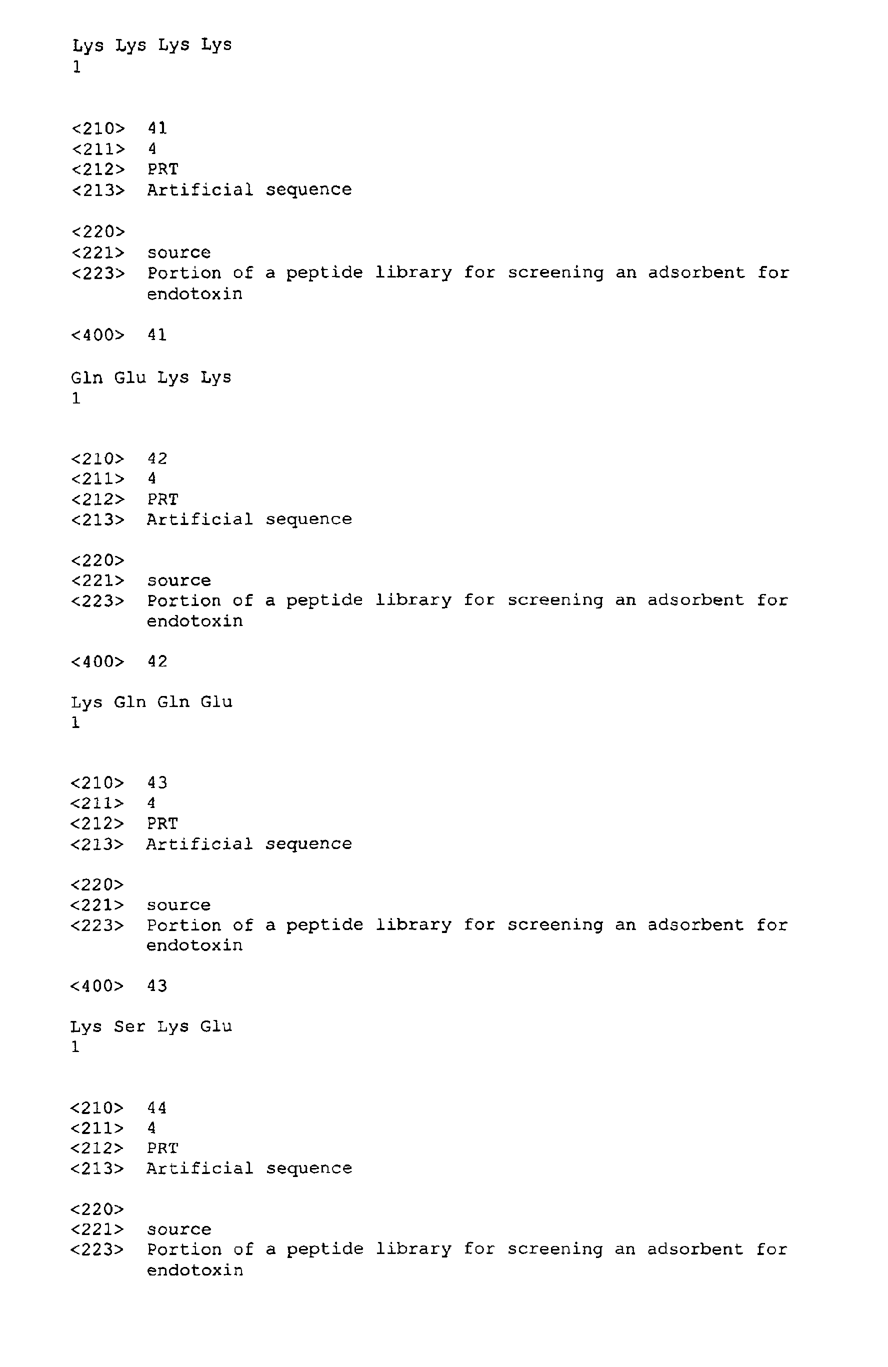

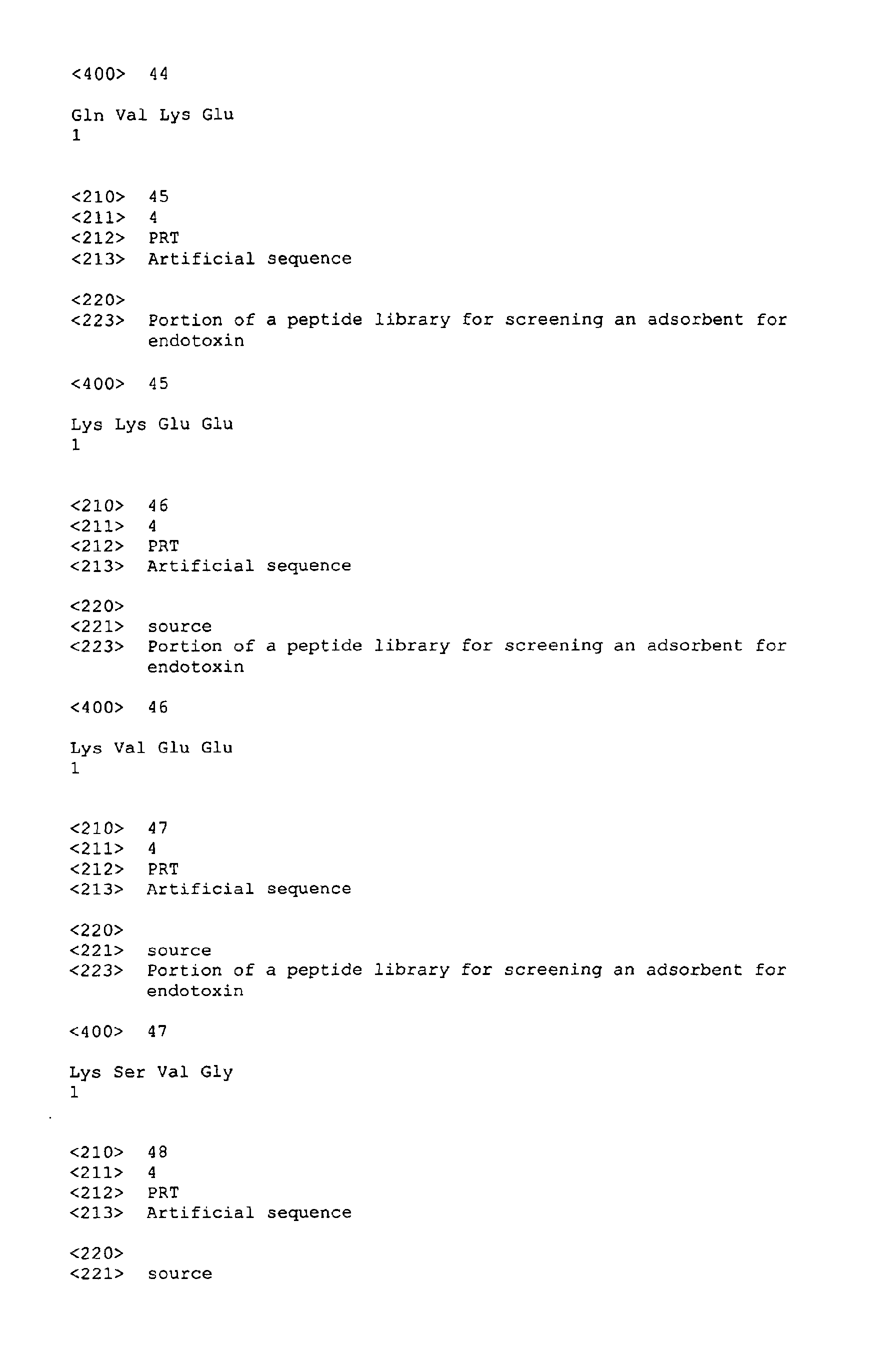

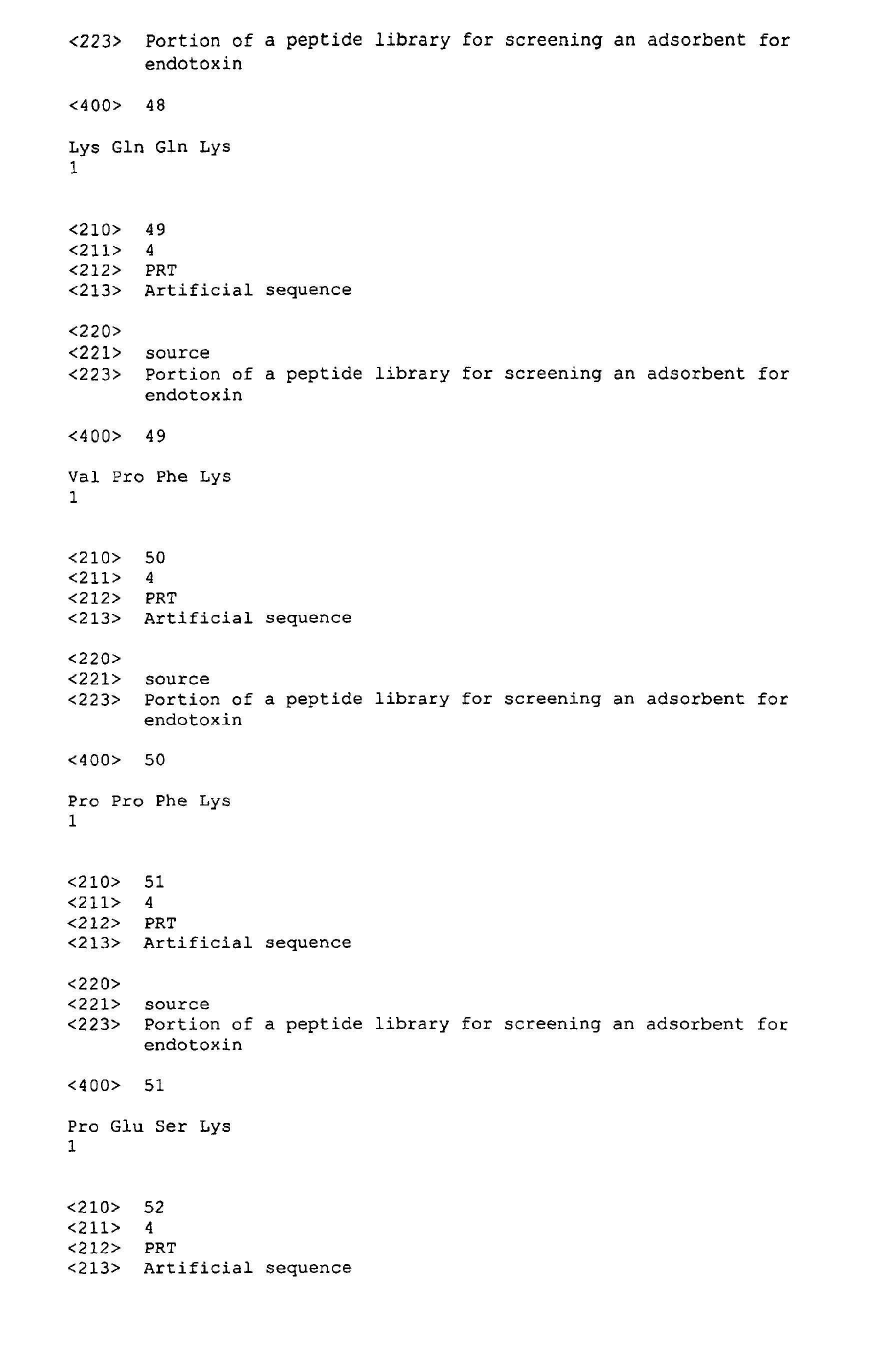

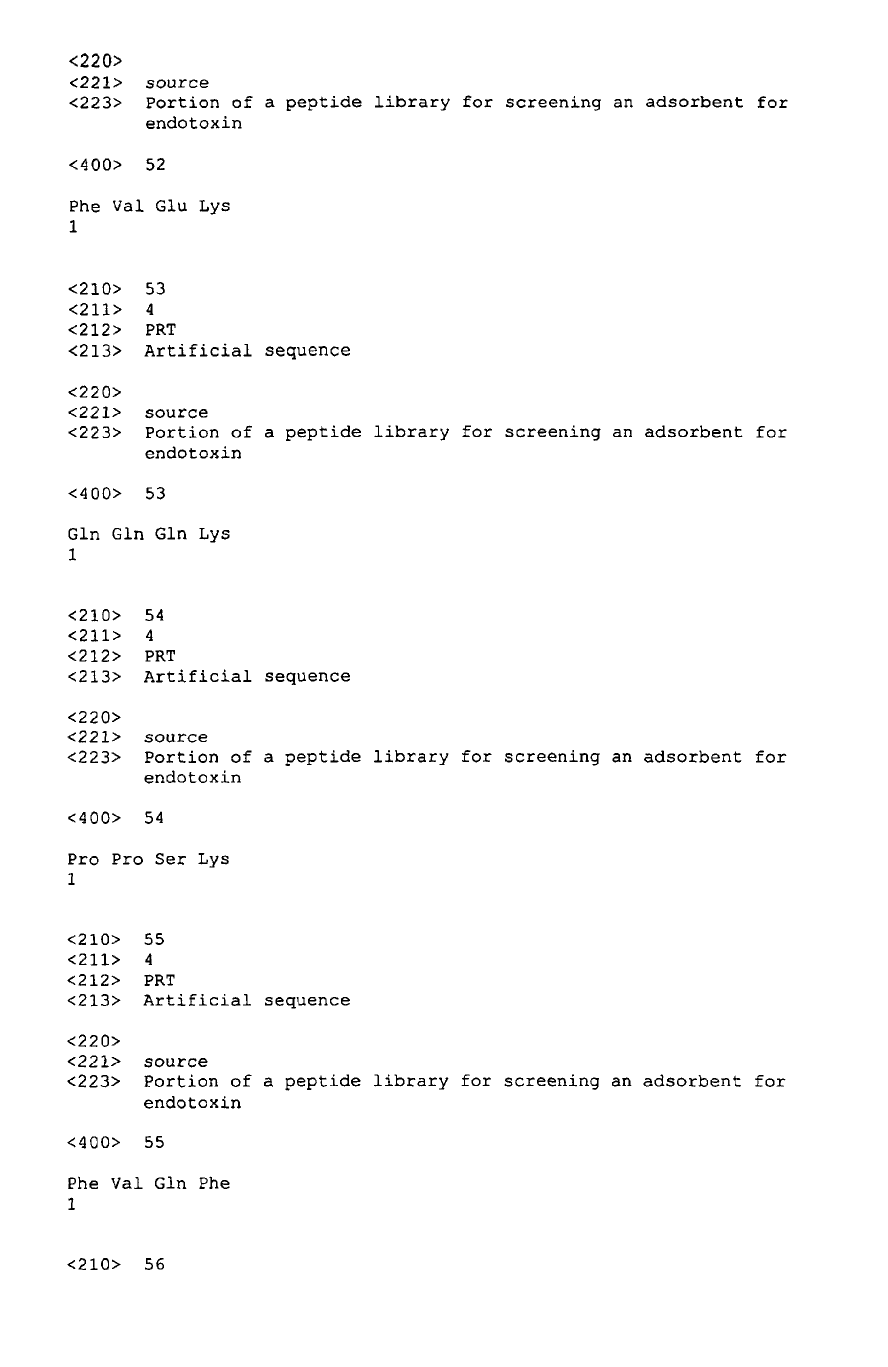

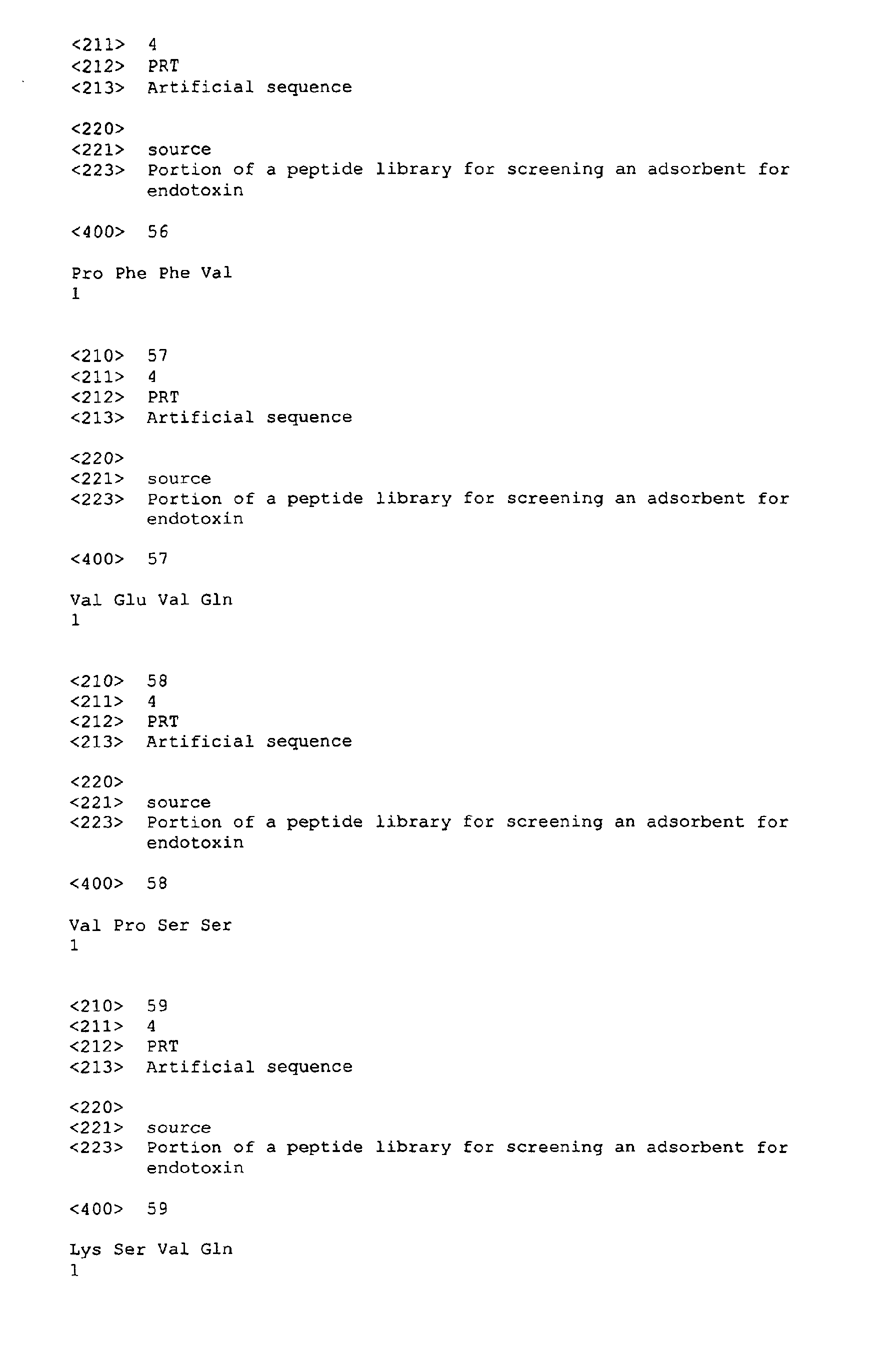

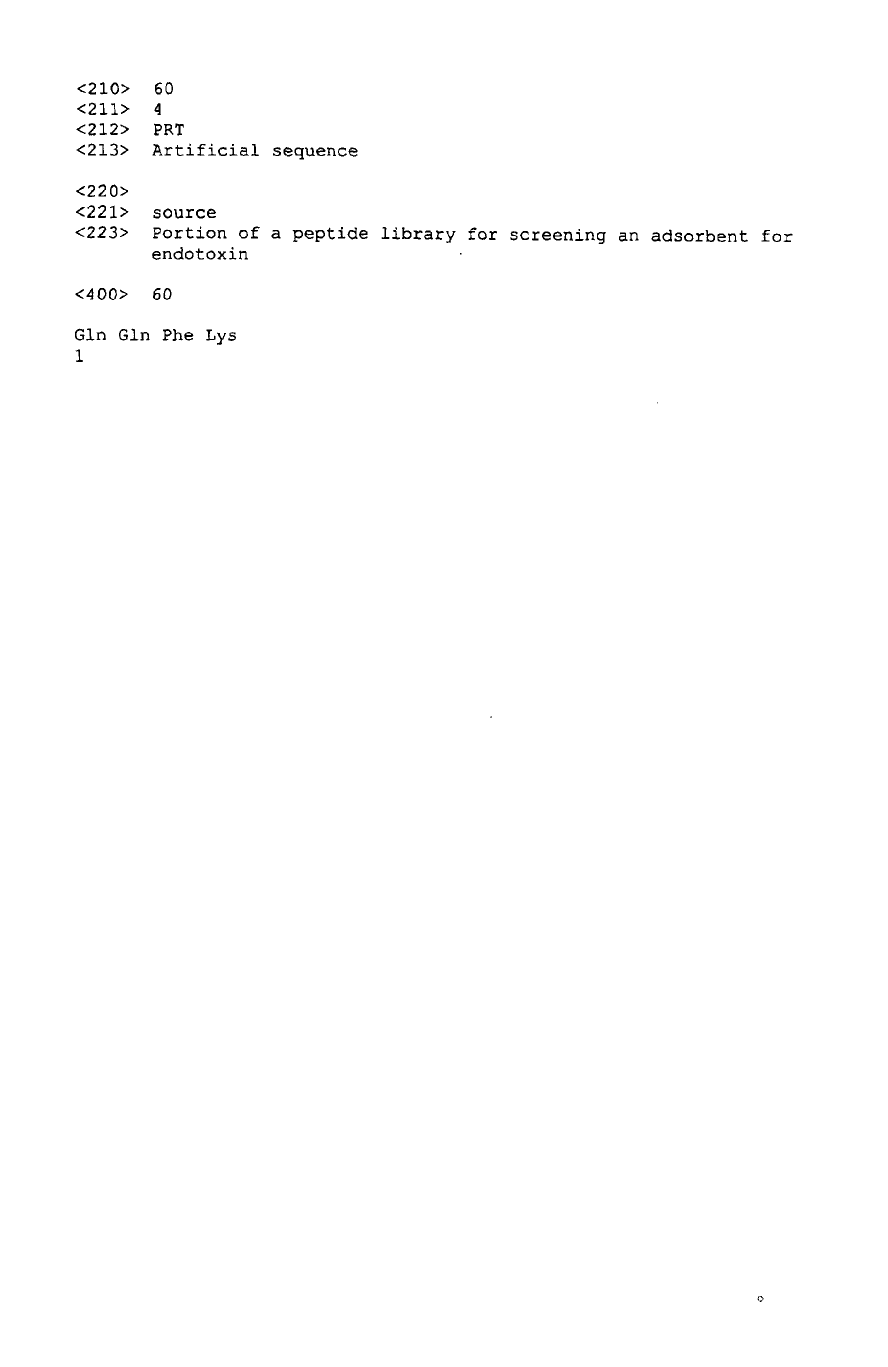

## Claims

1. A method for screening an adsorbent for endotoxin **characterized in that** a binding assay to a peptide library is carried out, using a lipid A radioisotope-labeled preparation where radioactive element is introduced into a phosphonoxyethyl derivative of lipid A of a biosynthetic precursor type or lipid A of an *Escherichia coli* type.

2. The method for screening according to claim 1, wherein the peptide library is a peptide library prepared in such a manner that a solid phase carrier or a soluble carrier having a functional group is bound to a compound, having a functional group which is able to bind to the said functional group and having two or more other functional groups, and an oligopeptide is further bound thereto.

3. The method for screening according to claim 1, wherein the peptide library is a peptide library prepared in such a manner that a solid phase carrier or a soluble carrier having a functional group is bound to a compound, having a functional group which is able to bind to the said functional group and having two or more other functional groups; further bound to a compound, having a functional group which is able to bind to the said other groups and having two or more other functional groups, and an oligopeptide is further bound thereto.

4. The method for screening according to claim 2 or 3, wherein the solid phase carrier or soluble carrier having a functional group is a solid phase carrier or a soluble carrier having an amino group.

5. The method for screening according to claim 2, wherein the solid phase carrier or soluble carrier having a functional group is a solid phase carrier or soluble carrier having an amino group, and the compound having a functional group which is able to bind to the said amino group and having two or more other functional groups is an amino acid represented by the formula HOOC(NH₂)CH(CH₂)ₙNH₂(in the formula, n is an integer of 1 to 4).

6. The method for screening according to claim 3, wherein the solid phase carrier or soluble carrier having a functional group is a solid phase carrier or soluble carrier having an amino group, and the compound having a functional group which is able to bind to the said amino group and having two or more other functional groups is an amino acid represented by the formula HOOC(NH₂)CH(CH₂)ₙNH₂(in the formula, n is an integer of 1 to 4), and further the compound having a functional group which is able to bind to the said other functional groups and having two or more still other functional groups is an amino acid represented by the formula HOOC(NH₂)CH(CH₂)ₙNH₂(in the formula, n is an integer of 1 to 4).

7. The method for screening according to claim 2, wherein the solid phase carrier or soluble carrier having a functional group is a solid phase carrier or soluble carrier having an amino group, and the compound having a functional group which is able to bind to the said amino group and having two or more other functional groups is lysine or ornithine.

8. The method for screening according to claim 3, wherein the solid phase carrier or soluble carrier having a functional group is a solid phase carrier or soluble carrier having an amino group, and the compound having a functional group which is able to bind to the said amino group and having two or more other functional groups is lysine or ornithine, and further the compound having a functional group which is able to bind to the said other functional groups and having two or more still other functional groups is lysine or ornithine.

9. The method for screening according to claim 2, wherein the solid phase carrier or soluble carrier having a functional group is a solid phase carrier or soluble carrier having an amino group, and the compound having a functional group which is able to bind to the said amino group and having two or more other functional groups is deoxycholic acid or chenodeoxycholic acid.

10. The method for screening according to claim 1, wherein a compound represented by the following formula [1] 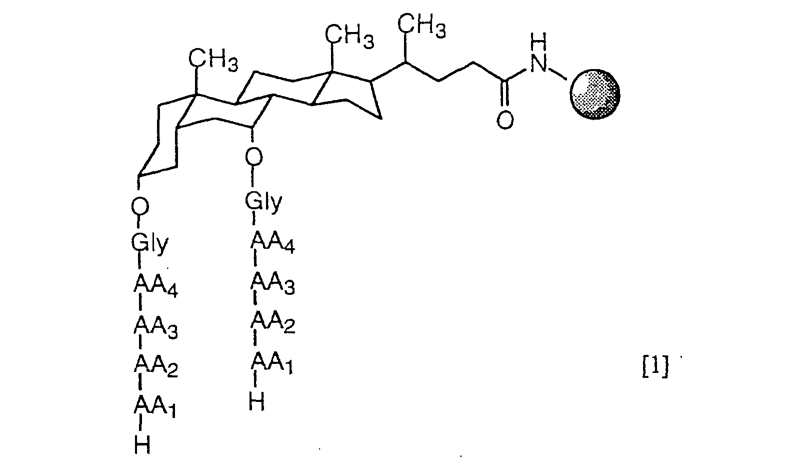 (in the formula, a spherical moiety represents a solid phase carrier or soluble carrier, and AA₁, AA₂, AA₃ and AA₄ each independently represents an amino acid residue) is used as a peptide library.

11. The method for screening according to claim 1, wherein a compound represented by the following formula [2] 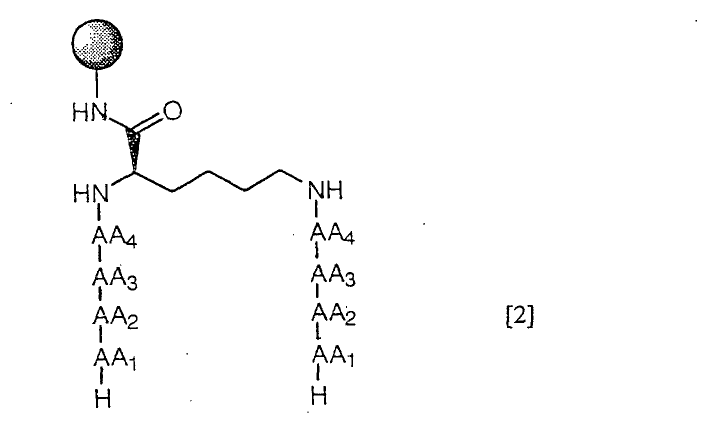 (in the formula, a spherical moiety represents a solid phase carrier or soluble carrier, and AA₁, AA₂, AA₃ and AA₄ each independently represents an amino acid residue) is used as a peptide library.

12. The method for screening according to claim 1, wherein a compound represented by the following formula [3] 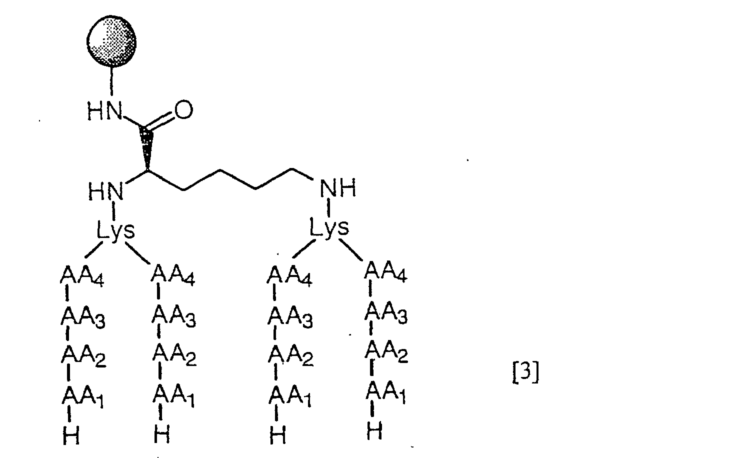 (in the formula, a spherical moiety represents a solid phase carrier or soluble carrier, and AA₁, AA₂, AA₃ and AA₄ each independently represents an amino acid residue) is used as a peptide library.

13. The method for screening according to any of claims 2 to 12, wherein the solid phase carrier is a resin in beads.

14. The method for screening according to any of claims 1 to 13, wherein the radioactive element is tritium (³H).

15. An adsorbent for endotoxin which is found by a method for screening mentioned in any of claims 1 to 14.

16. A peptide library in which a solid phase carrier or soluble carrier having a functional group is bound to a compound having a functional group which is able to bind to the said functional group and having two or more other functional groups, and further bound to an oligopeptide.

17. The peptide library according to claim 16, wherein it is represented by the following formula [1] 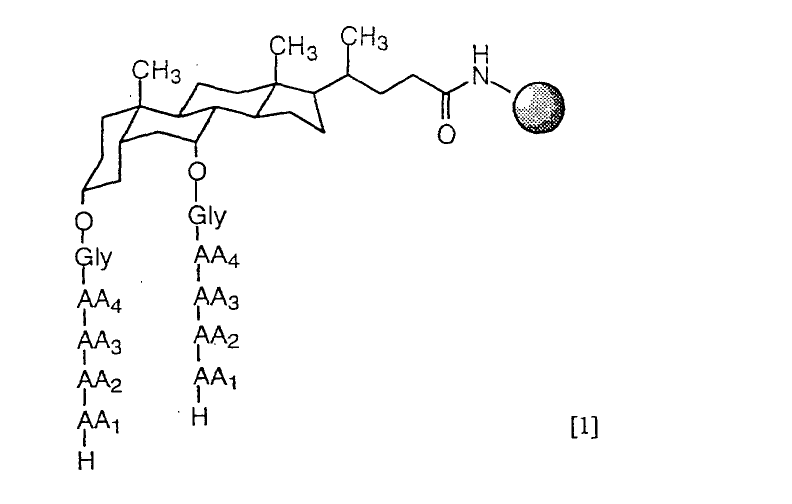 (in the formula, a spherical moiety represents a solid phase carrier or soluble carrier, and AA₁, AA₂, AA₃ and AA₄ each independently represents an amino acid residue).

18. The peptide library according to claim 17, wherein the spherical moiety in the formula [1] is a solid phase carrier.

19. The peptide library according to claim 18, wherein AA₁, AA₂, AA₃ and AA₄ in the formula [1] are any of the combinations mentioned below.
| AA1 | AA2 | AA3 | AA4 |
|---|---|---|---|
| D―Phe | D―Val | L―Gln | D―Phe |
| L―Lys | D―Ser | D―Val | D―Gln |
| D―Pro | D―Phe | L―Phe | L―Val |
| D―Gln | D―Gln | L―Phe | D―Lys |
| D―Val | L―Pro | D―Ser | D―Ser |

20. The peptide library according to claim 16, wherein it is represented by the following formula [2] 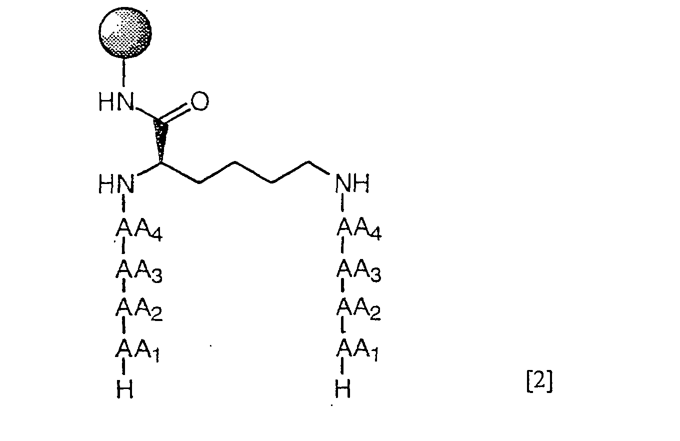 (in the formula, a spherical moiety represents a solid phase carrier or soluble carrier, and AA₁, AA₂, AA₃ and AA₄ each independently represents an amino acid residue).

21. The peptide library according to claim 20, wherein the spherical moiety in the formula [2] is a solid phase carrier.

22. The peptide library according to claim 21, wherein AA₁, AA₂, AA₃ and AA₄ in the formula [2] are any of the combinations mentioned below.
| AA1 | AA2 | AA3 | AA4 |
|---|---|---|---|
| D―Pro | L―Glu | D―Glu | L―Val |
| D―Val | D―Pro | D―Lys | L―Gln |
| D―Val | L―Glu | L―Val | D―Glu |
| L―Val | D―Pro | D―Val | D―Ser |
| L―Val | D―Pro | L―Phe | Gly |
| D―Lys | L―Glu | D―Glu | L―Gln |
| L―Lys | D―Lys | D―Lys | D―Ser |
| L―Phe | L―Lys | Gly | L―Ser |
| D―Gln | D―Glu | D―Ser | L―Glu |

23. A peptide library in which a solid phase carrier or soluble carrier having a functional group is bound to a compound having a functional group which is able to bind to the said functional group and having two or more other functional groups, then bound to a compound having functional groups which are able to bind to the said other functional groups and having two or more still other functional group, and further bound to an oligopeptide.

24. The peptide library according to claim 23, wherein it is represented by the following formula [3] 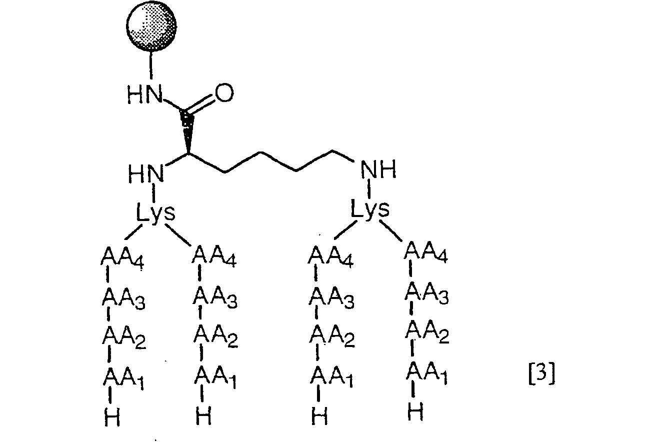 (in the formula, a spherical moiety represents a solid phase carrier or soluble carrier, and AA₁, AA₂, AA₃ and AA₄ each independently represents an amino acid residue).

25. The peptide library according to claim 24, wherein the spherical moiety in the formula [3] is a solid phase carrier.

26. The peptide library according to claim 25, wherein AA₁, AA₂, AA₃ and AA₄ in the formula [3] are any of the combinations mentioned below.
| AA1 | AA2 | AA3 | AA4 | AA1 | AA2 | AA3 | AA4 |
|---|---|---|---|---|---|---|---|
| D-Lys | D-Ser | D-Lys | L-Ser | D-Val | L-Glu | L-Val | L-Lys |
| L-Gln | D-Lys | L-Glu | L-Gln | D-Pro | L-Ser | D-Glu | L-Lys |
| D-Ser | L-Phe | D-Gln | L-Lys | D-Ser | L-Ser | L-Lys | D-Lys |
| L-Phe | D-Lys | L-Gln | L-Lys | L-Lys | L-Lys | D-Lys | D-Lys |
| L-Lys | L-Lys | L-Gln | L-Lys | L-Gln | L-Glu | L-Lys | D-Lys |
| L-Val | L-Val | L-Lys | L-Lys | L-Lys | L-Gln | L-Gln | D-Glu |
| L-Gln | D-Lys | Gly | L-Lys | L-Lys | L-Ser | L-Lys | L-Glu |
| L-Glu | D-Gln | D-Phe | L-Lys | D-Gln | D-Val | D-Lys | L-Glu |
| D-Gln | D-Glu | L-Phe | L-Lys | D-Lys | L-Lys | D-Glu | L-Glu |
| L-Ser | D-Ser | L-Phe | D-Val | L-Lys | D-Val | L-Glu | D-Glu |
| D-Pro | L-Ser | L-Pro | L-Val | L-Lys | L-Ser | L-Val | Gly |
| D-Pro | L-Pro | D-Ser | L-Phe | L-Lys | D-Gln | L-Gln | L-Lys |
| L-Pro | L-Pro | L-Ser | L-Val | D-Val | L-Pro | L-Phe | L-Lys |
| L-Pro | L-Val | L-Gln | D-Gln | L-Pro | D-Pro | D-Phe | L-Lys |
| Gly | D-Val | L-Lys | Gly | L-Pro | L-Glu | L-Ser | L-Lys |
| L-Phe | L-Val | L-Glu | D-Ser | L-Phe | D-Val | D-Glu | L-Lys |
| L-Phe | L-Gln | L-Lys | L-ser | L-Gln | D-Gln | L-Gln | D-Lys |
| D-Lys | L-Gln | L-Pro | D-Phe | L-Pro | L-Pro | L-Ser | D-Lys |
| D-Lys | D-Lys | D-Val | D-Gln | | | | |

27. The peptide library according to any of claims 17, 19 and 22, wherein AA₄ is a lysine residue.

28. An adsorbent for endotoxin containing the peptide library mentioned in claim 18.

29. An adsorbent for endotoxin containing the peptide library mentioned in claim 20.

30. An adsorbent for endotoxin containing the peptide library mentioned in claim 23.

31. A lipid A radioisotope-labeled preparation where radioactive element is introduced into a phosphonoxyethyl derivative of lipid A of a biosynthetic precursor type or lipid A of an *Escherichia coli* type.

32. The lipid A radioisotope-labeled preparation according to claim 31, wherein the lipid A radioisotope-labeled preparation, in which radioactive element is introduced into a phosphonoxyethyl derivative of lipid A of a biosynthetic precursor type, is represented by the following structural formula [4].

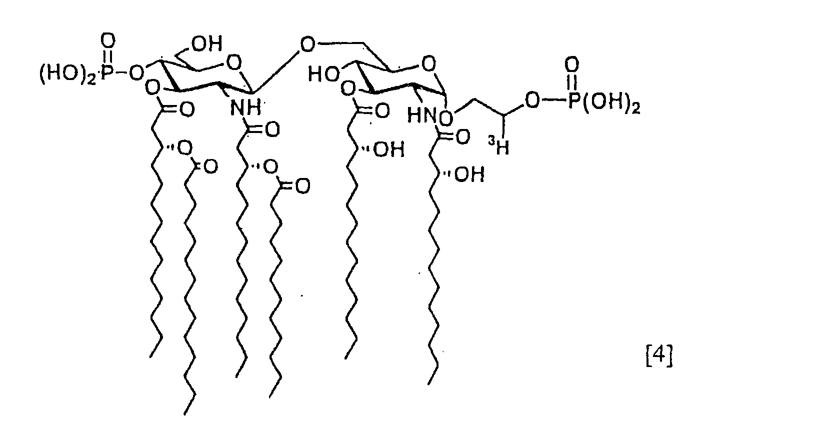

33. The lipid A radioisotope-labeled preparation according to claim 31, wherein the lipid A radioisotope-labeled preparation in which radioactive element is introduced into a phosphonoxyethyl derivative of lipid A of an *Escherichia coli* type is represented by the following structural formula [5].
